# EUROPEAN PATENT APPLICATION

(11) **EP 4 292 440 A1**
(43) Date of publication of application: **20.12.2023**
(21) Application number: 22179019.9
(22) Date of filing: 14.06.2022
(51) Int. Cl.: A23J 3/22, C12N 5/077, C12N 5/071

(54) **SUSPENSION BASED CHICKEN PRODUCT FORMULATION**

(71) Applicant: Upside Foods, Inc., Berkeley CA 94710 (US)
(72) Inventor: The designation of the inventor has not yet been filed
(74) Representative: Mewburn Ellis LLP

(57) **Abstract**

Provided herein are cell based meat compositions suitable for consumption comprising cells harvested from suspension cell culture and comprising an emulsion mixture. Also provide here are methods of making a cell based meat composition suitable for consumption comprising: culturing a population of cells in suspension; harvesting the population of cells to produce a cell mass; formulating an emulsion mixture by: (i) emulsifying an oil with a hydrocolloid to produce an oil:hydrocolloid mixture; and (ii) adding the cell mass to the oil:hydrocolloid mixture to form an emulsion mixture; and (d) forming the cell based meat composition into a patty.

## Description

### 1. BACKGROUND OF THE INVENTION

Foods formulated from metazoan cells, cultured *in vitro,* have prospective advantages over their corporal-derived animal counterparts, including improved nutrition and safety. Production of these products have been projected to require fewer resources, convert biomass at a higher caloric efficiency and result in reduced environmental impacts relative to conventional in vivo methods. Together, metazoan cells, and their extracellular products, constitute a biomass that can potentially be harvested from a cultivation infrastructure for formulation of cell-based food products, such as cultured meat.

However, several challenges persist including mass production of enough cells for formulating into a cell-based food product and the moisture content, texture profile, and flavor of the resulting cell-based food product formulations. Current production methods lack robustness in terms of generating enough cells. Moreover, current formulations of cell-based food products lack a moisture content, texture profile, and flavor similar enough to conventional meat to make formulations desirable among consumers.

### 2. SUMMARY OF THE INVENTION

This disclosure is based in part on the finding that cell based meat compositions suitable for consumption comprising cells (e.g., chicken cells) harvested from suspension culture and an emulsion mixture (e.g., an oil, a hydrocolloid, and cells, or a combination thereof) enable the cell based meat composition to exhibit a moisture content and/or texture profile (e.g., hardness) unique from other cell based meat compositions suitable for consumption and more similar to conventional meats. Applicant found that growing cells in suspension culture produces sufficient numbers and quality of cells needed for cell-based food product formulations. In addition, Applicant found that using an emulsion mixture, and in particular an emulsion mixture comprising a hydrocolloid (e.g., methylcellulose) enables a moisture content (e.g., as indicated by water activity (A_{w})) and a texture profile (e.g., hardness) unique from other cell based meat compositions suitable for consumption and more similar to conventional meat. Additionally, Applicant found that the methods for making the cell based meat compositions described herein do not require heat(ing) during production, let alone heating the cells above 40°C as currently required by the field. In contrast, in at least one embodiment, the methods for making the cell based meat compositions described herein maintain an internal temperature of the cell mass(es) and/or cell based meat composition at or below 5°C.

### 3. BRIEF DESCRIPTION OF THE DRAWINGS

These and other features, aspects, and advantages of the present invention will become better understood with regard to the following description, and accompanying drawings, where:
**FIG. 1** shows an example workflow for producing a cell based meat patty comprising chicken cells.
**FIG. 2** shows a schematic of a non-limiting example workflow for producing a cell based meat patty.

### 4. DETAILED DESCRIPTION OF THE INVENTION

Provided herein are cell based meat compositions suitable for consumption and methods of making the same. In particular, this disclosure is based on the finding that cell based meat compositions suitable for consumption comprising cells (e.g., chicken cells) harvested from suspension culture and an emulsion mixture (e.g., an oil, a hydrocolloid, and cells, or a combination thereof) enable the cell based meat composition to exhibit a moisture content and/or texture profile (e.g., hardness) unique from other cell based meat compositions suitable for consumption and more similar to conventional meats. In addition, Applicant found that growing cells in suspension culture produces sufficient numbers and quality of cells needed for cell-based food product formulations. Applicant also found that using an emulsion mixture, and in particular an emulsion mixture comprising a hydrocolloid (e.g., methylcellulose) in place of a cross-linking agent, enables a moisture content (e.g., as indicated by water activity (A_{w})) and a texture profile (e.g., hardness (i.e., Newtons)) unique from other cell based meat compositions suitable for consumption and more similar to conventional meat.

### 4.1. Definitions

Unless defined otherwise, all technical and scientific terms used herein have the same meaning as is commonly understood by one of ordinary skill in the art to which this disclosure belongs. In the event that there is a plurality of definitions for terms cited herein, those in this section prevail unless otherwise stated.

Throughout this disclosure, the term "a" or "an" entity refers to one or more of that entity. As such, the terms "a" (or "an"), "one or more," and "at least one" can be used interchangeably herein. Furthermore, "and/or" as used in a phase such as "A and/or B" herein is intended to include "A and B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

As used herein, the terms " "adhesive capability" refer to a TPA parameter that quantifies a material's tendency to adhere to the probe.

As used herein, the term "binding" as used herein refers to promoting, supporting, or enabling holding together ingredients in one cohesive mass. A method for quantifying binding is described in Example 3.

As used herein, the term "binding agent" refers to an agent that mediates binding.

As used herein, the term "chewiness" refers to a TPA parameter that is calculated as the product of the TPA parameters gumminess and springiness (see Example 3). Without wishing to be bound by theory, chewiness is thought to express the energy required to chew a food product to a state where it is ready for swallowing. Non-limiting variables that can be titrated to modulate the chewiness of the meat-like food products provided herein include but are not limited to densities of textured proteins, and moisture content.

As used herein, the term "cohesiveness" as used herein refers to a TPA parameter that is calculated from the area of work during the first compression of the cell based meat composition (see Example 3). Without wishing to be bound by theory, cohesiveness is thought to express the structural integrity of a food product, and refers to a property characterized by the strength of internal bonding that makes up the body of a cell based meat composition. Non-limiting variables that can be titrated to modulate the cohesiveness of the meat-like cell based meat composition provided herein include but are not limited to types and amounts of binding agents.

As used herein, the terms "comprising" and "including" or grammatical variants thereof are to be taken as specifying the stated features, integers, steps, or components but do not preclude the addition of one or more additional features, integers, steps, components, or groups thereof. This term encompasses the terms "consisting of" and "consisting essentially of'.

As used herein, the terms "cell" and "cell line" are sometimes used interchangeably. As used herein, the term "cell" can refer to one or more cells originating from a cell line. As used herein, the term "cell line" can refer to a population of cells.

As used herein, the terms "cell mass" or "hydrated cell mass" refer to a population of cells harvested from suspension culture.

As used herein, the phrases "cell based meat composition," "cell-based meat," "slaughter-free meat," "slaughter-free cell-based meat," "in vitro produced meat," " in vitro cell-based meat," "cultured meat," "slaughter-free cultured meat," "in vitro produced cultured meat," "in vitro meat," "in vitro cultured meat" and other similar such phrases are interchangeably used herein, and refer to the meat that is generated in vitro, starting with cells in culture, and that method which does not involve the slaughter of an animal in order to directly obtain meat from that animal for dietary consumption.

As used herein, the term "cultivation infrastructure" refers to the environment in which liver-derived cells, dedifferentiated cell, or myogenic cells (e.g., non-naturally occurring myogenic cells) are cultured (i.e., the environment in which the myogenic cell is cultivated).

As used herein, the term "dry blend" refers to a mixture of one or more ingredients substantially free of a solution.

As used herein, the term "dry weight" refers to weight after drying. Drying can be accomplished by any means that allows calculation of weight after drying.

As used herein, the term "emulsion" refers to a mixture of immiscible liquids in which one or more liquids ("dispersed phase(s)") are dispersed as fine droplets in another liquid ("continuous phase").

As used herein, the term "emulsifier" refers to a molecule that concentrates at the interface between the phases of an emulsion and reduces the interfacial tension between the phases and thus stabilizes the emulsion.

As used herein, the terms "emulsion mixture" or "emulsion subcomponent" refer to a combination of reagents selected from an oil, a hydrocolloid, and a cell mass, or a combination thereof. In some cases, the "emulsion mixture" or "emulsion subcomponent" are added to the "hydrated textured subcomponent," a "dry blend" or a combination thereof to produce a cell based meat composition. In some cases, the "emulsion mixture" or "emulsion subcomponent" are added to a cell mass (e.g., a population of cells harvested from suspension culture) to produce a cell based meat composition.

As used herein, the term "gumminess" refers to a Texture Profile Analysis (TPA) parameter of a cell based meat composition and is the product of the TPA parameters hardness and cohesiveness (see Example 3).

As used herein, the term "hardness" refers to a texture parameter of a food product and is calculated from the peak force of the first compression of the cell based meat composition in either a TPA assay (see example 3) or a compression assay. Without wishing to be bound by theory, "hardness" is thought to correlate with the force required to compress a cell based meat composition between molars during chewing. Non-limiting variables that can be titrated to modulate the hardness of the cell based meat compositions provided herein include but are not limited to oil, hydrocolloid content, cell mass content (e.g., first and/or second hydrated cell masses) textured protein products with different densities, moisture content, and pH.

As used herein, the terms "hydration mixture" or "hydration subcomponent" refer to a combination of reagents selected from a cell mass, water, and one or more flavoring agents, or a combination thereof. In some cases, the "hydration mixture" or "hydration subcomponent" are added to one or more textured protein to produce a "hydrated textured subcomponent."

As used herein, the term "hydrated textured subcomponent" refers to the product of mixing the "hydration mixture" or "hydration subcomponent" with one or more textured proteins.

As used herein, the term "hydrated" refers to the presence of one or more solutions. The term "hydrating" refers to the addition of one or more solutions to a reagent, ingredient, or mixture of ingredients. The term "hydration" refers to the process where a reagent, ingredient or mineral structure in contact with a solution absorbs the solution as a result of the hydrophilic nature of the reagent, ingredient, or mixture of ingredients. The rate of hydration can be determined by the particle size, morphology, chemical structure, and molecular weight of the reagent, ingredient, or mixture of ingredients. The terms "hydrated cell mass," "hydrated first cell mass," and "hydrated second cell mass" refer to the presence of one or more solutions along with the cell mass, first cell mass, or second cell mass, respectively.

As used herein, the term "hydrocolloid" refers to a heterogeneous group of long chain polymers (polysaccharides and proteins) characterized by their property of forming viscous dispersions and/or gels when dispersed in water. Without wishing to be bound by theory, the presence of a large number of hydroxyl (-OH) groups in the hydrocolloids markedly increases their affinity for binding water molecules, rendering them hydrophilic compounds. In some cases, hydrocolloids are emulsifiers and/or emulsion stabilizers. In some cases, hydrocolloids are binding agents.

As used herein, the term "ingredients" refers to, without limitation, biologically active substances, food safe ingredients, non-animal ingredients, animal ingredients, nutritional supplements, flavoring agents, salts, sugars, vitamins, and minerals used in the cell based meat compositions described herein.

As used herein, the term "myogenic" refers to a cell that has characteristics of a skeletal muscle tissue. Natively myogenic cells include, but are not limited to, myoblasts, myocytes, satellite cells, side population cells, muscle derived stem cells, mesenchymal stem cells, myogenic pericytes, or mesoangioblasts

As used herein, the term "moisture content" refers to the amount of water in a food product calculated as a percentage change in mass following the evaporation of water from a sample. Variables that can be titrated to modulate the moisture content of the cell based meat composition provided herein include but are not limited to textured proteins with different water absorbing characteristics, lipid content, and/or binding agents with different water retention characteristics.

As used herein, unless otherwise indicated, the term "percent," "percentage," or "%" refers to percent, percentage, or "%" of total.

As used herein, the term "resilience" refers to a TPA parameter of a cell based meat composition and is calculated by dividing the upstroke energy of the first compression by the downstroke energy of the first compression (see Example 3). Without wishing to be bound by theory, resilience is thought to express how well a cell based meat composition fights to regain its original shape.

As used herein, the term "springiness" refers to a TPA parameter of a cell based meat composition and is calculated as the ratio of the cell based meat composition's height during the second compression and the original compression distance (see Example 3). Without wishing to be bound by theory, springiness is thought to correlate with the ability of a cell based meat composition to spring back after deformation.

As used herein, the term "substantially free of" or "substantially free from" means the amount (e.g., absolute number within a population or concentration/percentage within a population) of a cell or cell type is below a value where the cell or cell type, or any cell derived therefrom, could contribute to the population. For example, a population substantially free of a cell means that upon differentiation of the population the cell does not contribute progeny to the differentiated population.

As used herein, the term "suspension culture" or "cell suspension culture" refers to a type of culture in which single cells or small aggregates of cells are cultured as non-adherent cells or aggregates of cells.

As used herein, the term "texture" refers to mechanical characteristics of a cell based meat composition that are correlated with sensory perceptions of the cell based meat composition.

As used herein, the terms "texture profile analysis" or "TPA" refers to the double compression test or similar test for determining the textural properties of a cell based meat composition. A TPA test uses multiple rounds of compression as a texture analyzer to provide insight into how samples behave when chewed. In some cases, TPA quantifies hardness, cohesiveness, springiness, and resilience. See Example 3 for an example TPA method.

As used herein, the term "textured protein" refers to a textured protein used as a meat analogue or meat extender. A non-limiting example of a "textured protein" is a textured soy protein (TSP), which can be referred to as soy meat or soya chunks. Textured protein can be produced from any protein-rich seed meal.

As used herein, the term "water activity" or "(a_{w})" refers to the hygroscopic nature of a substance; or the tendency of a substance that absorbs water from its surroundings. The water activity of a solution is expressed as Aw=P/Po, where P is the water vapor pressure of the solution and Po is the vapor pressure of pure water at the same temperature; water activity is the partial vapor pressure of water in a solution divided by the standard state partial vapor pressure of water. In the field of food science, the standard state is most often defined as pure water at the same temperature. A water activity of 0.80 means the vapor pressure is 80 percent of that of pure water. The water activity increases with temperature. The moisture condition of a product can be measured as the equilibrium relative humidity (ERH) expressed in percentage or as the water activity expressed as a decimal.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

### 4.2. Cell Based Meat Composition

Provided herein are cell based meat compositions or food products comprising poultry cells (e.g., fibroblasts, myofibroblasts, and myogenic cells). In some embodiments, the poultry cells (e.g., fibroblasts, myofibroblasts, and myogenic cells) are combined with other ingredients to make a cell based meat product (e.g., a patty. In some embodiments, the poultry cells (e.g., fibroblasts, myofibroblasts, and myogenic cells) are combined with an emulsion mixture to make a cell based meat product (e.g., a patty).

The cell based meat compositions provided herein meet standards for food safety required by government regulation. In some embodiments, the cell based meat compositions meet standards for food safety required by the U.S. Food and Drug Administration (FDA), the U.S. Department of Agriculture (USDA), the European Food Safety Authority, or other state or regional food regulatory agencies. In such embodiments, the cell based meat compositions include ingredients that meet standards for food safety required by the FDA, USDA, and European Food Safety Authority. In some embodiments, the cell based meat compositions may comprise ingredients approved for use in food that are recognized by the FDA as Generally Recognized As Safe (GRAS). In some embodiments, the cell based meat compositions comprises ingredients that are GRAS certified ingredients, non-GRAS certified ingredients, or mixtures thereof. In some embodiments, the cell based meat composition comprises only GRAS certified ingredients. As the list of GRAS substances is updated by the FDA, the GRAS certified ingredients that may be used in the cell based meat composition may be modified.

In one aspect, provided herein is a cell based meat composition suitable for consumption, where the cell based meat composition comprises: about 5% to about 70% by weight of a hydration mixture, wherein the hydration mixture comprises a hydrated first cell mass, wherein the hydrated first cell mass comprises of a population of cells harvested from suspension culture; about 11% to about 33% by weight of a textured protein; about 1% to about 12% by weight of a dry blend; about 5.0% to about 70% by weight of an emulsion mixture, wherein the emulsion mixtures comprises an oil, a hydrocolloid and a hydrated second cell mass; wherein the cell based meat patty comprises a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

### 4.2.1. Hydration Mixture

In some embodiments, the cell based meat composition comprises a hydration mixture. In some embodiments, the hydration mixture comprises a hydrated first cell mass which comprises a population of cells harvested from suspension culture, a water, and a flavoring agent. In some embodiments, the hydration mixture also includes a second flavoring agent. In some embodiments, the hydration mixture was used, in part, to hydrate one or more textured proteins. In such embodiments, the moisture from the hydration mixture was the primary source of the moisture in the mixture that resulted.

In some embodiments, the cell based meat composition suitable for consumption comprises about 5% to about 70% (e.g., about 5% to about 65%, about 5% to about 60%, about 5% to about 55%, about 5% to about 50%, about 5% to about 45%, about 5% to about 45%, about 5% to about 40%, about 5% to about 35%, about 5% to about 30%, about 5% to about 25%, about 5% to about 20%, about 5% to about 15%, about 5% to about 10%, about 10% to about 70%, about 10% to about 65%, about 10% to about 60%, about 10% to about 55%, about 10% to about 50%, about 10% to about 45%, about 10% to about 40%, about 10% to about 35%, about 10% to about 30%, about 10% to about 25%, about 10% to about 20%, about 10% to about 15%, about 15% to about 70%, about 15% to about 65%, about 15% to about 60%, about 15% to about 55%, about 15% to about 50%, about 15% to about 45%, about 15% to about 40%, about 15% to about 35%, about 15% to about 30%, about 15% to about 25%, about 15% to about 20%, about 20% to about 70%, about 20% to about 65%, about 20% to about 60%, about 20% to about 55%, about 20% to about 50%, about 20% to about 45%, about 20% to about 40%, about 20% to about 35%, about 20% to about 30%, about 20% to about 25%, about 25% to about 70%, about 25% to about 65%, about 25% to about 60%, about 25% to about 55%, about 25% to about 50%, about 25% to about 45%, about 25% to about 40%, about 25% to about 35%, about 25% to about 30%, about 30% to about 70%, about 30% to about 65%, about 30% to about 60%, about 30% to about 55%, about 30% to about 50%, about 30% to about 45%, about 30% to about 40%, about 30% to about 35%, about 35% to about 70%, about 35% to about 65%, about 35% to about 60%, about 35% to about 55%, about 35% to about 50%, about 35% to about 45%, about 35% to about 40%, about 40% to about 70%, about 40% to about 65%, about 40% to about 60%, about 40% to about 55%, about 40% to about 50%, about 40% to about 45%, about 45% to about 70%, about 45% to about 65%, about 45% to about 60%, about 45% to about 55%, about 45% to about 50%, about 50% to about 70%, about 50% to about 65%, about 50% to about 60%, about 50% to about 55%, about 55% to about 70%, about 55% to about 65%, about 55% to about 60%, about 60% to about 70%, about 60% to about 65%, or about 65% to about 70%) by weight of a hydration mixture (e.g., a hydration mixture that comprises a hydrated first cell mass which comprises a population of cells harvested from suspension culture).

In some embodiments, the cell based meat composition suitable for consumption comprises at least 0.5%, at least 1%, at least 2%, at least 3%, at least 4%, at least 5%, at least 6%, at least 7%, at least 8%, at least 9%, at least 10%, at least 11%, at least 12%, at least 14%, at least 15%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 45%, at least 46%, at least 47%, at least 48%, at least 49%, at least 50%, at least 55%, at least 60%, at least 65%, at least 70%, at least 75%, at least 80%, at least 85 %, at least 90%, or at least 95% by weight of a hydration mixture (e.g., a hydration mixture that comprises a hydrated first cell mass which comprises a population of cells harvested from suspension culture).

In some embodiments, the hydration mixture comprises: about 60% to about 80% by weight of water; about 10% to about 30% by weight of the cell mass; about 1.5% to about 4.5% by weight of a first flavoring agent (e.g., a salt); and about 3.5% to about 10.5% by weight of a second flavoring agent (e.g., a chicken white meat flavor and/or a chicken breast flavor).

In some embodiments, the hydration mixture comprises a viscosity of about 1 cps to about 10,000 cps.

### 4.2.1.1 Water

In some embodiments, the hydration mixture comprises water. In some embodiments, the water is provided as part of the hydrated first cell mass. For example, a population of cells harvested from suspension culture comprises a dry cell pellet that is substantially free of solution and the dry cell pellet is hydrated using a solution (e.g., a water) to produce a hydrated cell mass (e.g., a hydrated first cell mass). In some embodiments, the water in the hydrated first cell mass is the primary source of water for the hydration mixture.

In some embodiments, the hydration mixture comprises about 60% to about 80% (e.g., about 60% to about 79%, about 60% to about 78%, about 60% to about 77%, about 60% to about 76%, about 60% to about 75%, about 60% to about 74%, about 60% to about 73%, about 60% to about 72%, about 60% to about 71%, about 60% to about 70%, about 60% to about 69%, about 60% to about 68%, about 60% to about 67%, about 60% to about 66%, about 60% to about 65%, about 60% to about 64%, about 60% to about 63%, about 60% to about 62%, about 60% to about 61%, about 61% to about 80%, about 61% to about 79%, about 61% to about 78%, about 61% to about 77%, about 61% to about 76%, about 61% to about 75%, about 61% to about 74%, about 61% to about 73%, about 61% to about 72%, about 61% to about 71%, about 61% to about 70%, about 61% to about 69%, about 61% to about 68%, about 61% to about 67%, about 61% to about 66%, about 61% to about 65%, about 61% to about 64%, about 61% to about 63%, about 61% to about 62%, about 62% to about 80%, about 62% to about 79%, about 62% to about 78%, about 62% to about 77%, about 62% to about 76%, about 62% to about 75%, about 62% to about 74%, about 62% to about 73%, about 62% to about 72%, about 62% to about 71%, about 62% to about 70%, about 62% to about 69%, about 62% to about 68%, about 62% to about 67%, about 62% to about 66%, about 62% to about 65%, about 62% to about 64%, about 62% to about 63%, about 63% to about 80%, about 63% to about 79%, about 63% to about 78%, about 63% to about 77%, about 63% to about 76%, about 63% to about 75%, about 63% to about 74%, about 63% to about 73%, about 63% to about 72%, about 63% to about 71%, about 63% to about 70%, about 63% to about 69%, about 63% to about 68%, about 63% to about 67%, about 63% to about 66%, about 63% to about 65%, about 63% to about 64%, about 64% to about 80%, about 64% to about 79%, about 64% to about 78%, about 64% to about 77%, about 64% to about 76%, about 64% to about 75%, about 64% to about 74%, about 64% to about 73%, about 64% to about 72%, about 64% to about 71%, about 64% to about 70%, about 64% to about 69%, about 64% to about 68%, about 64% to about 67%, about 64% to about 66%, about 64% to about 65%, about 65% to about 80%, about 65% to about 79%, about 65% to about 78%, about 65% to about 77%, about 65% to about 76%, about 65% to about 75%, about 65% to about 74%, about 65% to about 73%, about 65% to about 72%, about 65% to about 71%, about 65% to about 70%, about 65% to about 69%, about 65% to about 68%, about 65% to about 67%, about 65% to about 66%, about 66% to about 80%, about 66% to about 79%, about 66% to about 78%, about 66% to about 77%, about 66% to about 76%, about 66% to about 75%, about 66% to about 74%, about 66% to about 73%, about 66% to about 72%, about 66% to about 71%, about 66% to about 70%, about 66% to about 69%, about 66% to about 68%, about 66% to about 67%, about 67% to about 80%, about 67% to about 79%, about 67% to about 78%, about 67% to about 77%, about 67% to about 76%, about 67% to about 75%, about 67% to about 74%, about 67% to about 73%, about 67% to about 72%, about 67% to about 71%, about 67% to about 70%, about 67% to about 69%, about 67% to about 68%, about 67% to about 67%, about 68% to about 80%, about 68% to about 79%, about 68% to about 78%, about 68% to about 77%, about 68% to about 76%, about 68% to about 75%, about 68% to about 74%, about 68% to about 73%, about 68% to about 72%, about 68% to about 71%, about 68% to about 70%, about 68% to about 69%, about 69% to about 80%, about 69% to about 79%, about 69% to about 78%, about 69% to about 77%, about 69% to about 76%, about 69% to about 75%, about 69% to about 74%, about 69% to about 73%, about 69% to about 72%, about 69% to about 71%, about 69% to about 70%, about 70% to about 80%, about 70% to about 79%, about 70% to about 78%, about 70% to about 77%, about 70% to about 76%, about 70% to about 75%, about 70% to about 74%, about 70% to about 73%, about 70% to about 72%, about 70% to about 71%, about 71% to about 80%, about 71% to about 79%, about 71 % to about 78%, about 71% to about 77%, about 71 % to about 76%, about 71% to about 75%, about 71% to about 74%, about 71% to about 73%, about 71 % to about 72%, about 72% to about 80%, about 72% to about 79%, about 72% to about 78%, about 72% to about 77%, about 72% to about 76%, about 72% to about 75%, about 72% to about 74%, about 72% to about 73%, about 73% to about 80%, about 73% to about 79%, about 73% to about 78%, about 73% to about 77%, about 73% to about 76%, about 73% to about 75%, about 73% to about 74%, about 74% to about 80%, about 74% to about 79%, about 74% to about 78%, about 74% to about 77%, about 74% to about 76%, about 74% to about 75%, about 75% to about 80%, about 75% to about 79%, about 75% to about 78%, about 75% to about 77%, about 75% to about 76%, about 76% to about 80%, about 76% to about 79%, about 76% to about 78%, about 76% to about 77%, about 77% to about 80%, about 77% to about 79%, about 77% to about 78%, about 78% to about 80%, about 78% to about 79%, or about 79% to about 80%) by weight of water.

In some embodiments, the hydration mixture comprises at least 0.5%, at least 1%, at least 2%, at least 3%, at least 4%, at least 5%, at least 6%, at least 7%, at least 8%, at least 9%, at least 10%, at least 11%, at least 12%, at least 13% at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95% by weight of water.

### 4.2.1.2 First Cell Mass

In some embodiments, the hydration mixture includes a first cell mass which comprises a population of cells harvested from suspension culture. In some embodiments, the population of cells is harvested from *in vitro* in suspension culture. In some embodiments, the population of cells is separated from the components of the suspension culture. Separation can be achieved using filtration, centrifugation, gravity, or a combination thereof. Once separated from the components of the suspension culture (e.g., the cell culture medium), the population of cells may be a dry cell pellet. The cell pellet is hydrated with a solution to produce the hydrated first cell mass. Non-limiting examples of a solution include: water, saline solution, enrichment media, serum-free cell culture medium, and conditioned (filtered) medium from the suspension culture.

In some embodiments, the first cell mass (e.g., the hydrated first cell mass) comprises about 1.0 ×10⁶ to about 1× 10¹¹ (e.g., about 1.0 ×10⁶ to about 1× 10¹⁰, about 1.0 ×10⁶ to about 1× 10⁹, about 1.0 ×10⁶ to about 1× 10⁸, about 1.0 ×10⁶ to about 1× 10⁷, about 1.0 ×10⁷ to about 1× 10¹¹, about 1.0 ×10⁷ to about 1× 10¹⁰, about 1.0 ×10⁷ to about 1× 10⁹, about 1.0 ×10⁷ to about 1× 10⁸, about 1.0 ×10⁸ to about 1× 10¹¹, about 1.0 ×10⁸ to about 1× 10¹⁰, about 1.0 ×10⁸ to about 1× 10⁹, about 1.0 ×10⁹ to about 1× 10¹¹, about 1.0 ×10⁹ to about 1× 10¹⁰, or about 1.0 ×10¹⁰ to about 1× 10¹¹) cells.

In some embodiments, the first cell mass (e.g., the hydrated first cell mass) comprises a packed cell volume (PCV) of at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least, 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95%. Packed cell volume (PCV) refers to the percentage of the suspension solution that comprises cells.

In some embodiments, the dry weight of the first cell mass (e.g., the population of cells harvested from the suspension culture) comprises at least about 0.001 grams (g), at least about 0.005 g, at least about 0.01 g, at least about 0.05 g, at least about 0.1 g, at least about 0.5 g, at least about 1.0 g, at least about 2.0 g, at least about 3.0 g, at least about 4.0 g, at least about 5.0 g, at least about 6.0 g, at least about 7.0 g, at least about 8.0 g, at least about 9.0 g, at least about 10.0 g, at least about 11 g, at least about 12 g, at least about 13 g, at least about 14 g, at least about 15 g, at least about 16 g, at least about 17 g, at least about 18 g, at least about 19 g, at least about 20 g, at least about 21 g, at least about 22 g, at least about 23 g, at least about 24 g, at least about 25 g, at least about 26 g, at least about 27 g, at least about 28 g, at least about 29 g, or at least about 30 g.

In some embodiments, the hydration mixture comprises about 10% to about 30% (e.g., about 10% to about 29%, about 10% to about 28%, about 10% to about 27%, about 10% to about 26%, about 10% to about 25%, about 10% to about 24%, about 10% to about 23%, about 10% to about 22%, about 10% to about 21%, about 10% to about 20%, about 10% to about 19%, about 10% to about 18%, about 10% to about 17%, about 10% to about 16%, about 10% to about 15%, about 10% to about 14%, about 10% to about 13%, about 10% to about 12%, about 10% to about 11%, about 11% to about 30%, about 11% to about 29%, about 11% to about 28%, about 11% to about 27%, about 11% to about 26%, about 11% to about 25%, about 11% to about 24%, about 11% to about 23%, about 11% to about 22%, about 11% to about 21%, about 11% to about 20%, about 11% to about 19%, about 11% to about 18%, about 11% to about 17%, about 11% to about 16%, about 11% to about 15%, about 11% to about 14%, about 11% to about 13%, about 11% to about 12%, about 12% to about 30%, about 12% to about 29%, about 12% to about 28%, about 12% to about 27%, about 12% to about 26%, about 12% to about 25%, about 12% to about 24%, about 12% to about 23%, about 12% to about 22%, about 12% to about 21%, about 12% to about 20%, about 12% to about 19%, about 12% to about 18%, about 12% to about 17%, about 12% to about 16%, about 12% to about 15%, about 12% to about 14%, about 12% to about 13%, about 13% to about 30%, about 13% to about 29%, about 13% to about 28%, about 13% to about 27%, about 13% to about 26%, about 13% to about 25%, about 13% to about 24%, about 13% to about 23%, about 13% to about 22%, about 13% to about 21%, about 13% to about 20%, about 13% to about 19%, about 13% to about 18%, about 13% to about 17%, about 13% to about 16%, about 13% to about 15%, about 13% to about 14%, about 14% to about 30%, about 14% to about 29%, about 14% to about 28%, about 14% to about 27%, about 14% to about 26%, about 14% to about 25%, about 14% to about 24%, about 14% to about 23%, about 14% to about 22%, about 14% to about 21%, about 14% to about 20%, about 14% to about 19%, about 14% to about 18%, about 14% to about 17%, about 14% to about 16%, about 14% to about 15%, about 15% to about 30%, about 15% to about 29%, about 15% to about 28%, about 15% to about 27%, about 15% to about 26%, about 15% to about 25%, about 15% to about 24%, about 15% to about 23%, about 15% to about 22%, about 15% to about 21%, about 15% to about 20%, about 15% to about 19%, about 15% to about 18%, about 15% to about 17%, about 15% to about 16%, about 16% to about 30%, about 16% to about 29%, about 16% to about 28%, about 16% to about 27%, about 16% to about 26%, about 16% to about 25%, about 16% to about 24%, about 16% to about 23%, about 16% to about 22%, about 16% to about 21%, about 16% to about 20%, about 16% to about 19%, about 16% to about 18%, about 16% to about 17%, about 17% to about 30%, about 17% to about 29%, about 17% to about 28%, about 17% to about 27%, about 17% to about 26%, about 17% to about 25%, about 17% to about 24%, about 17% to about 23%, about 17% to about 22%, about 17% to about 21%, about 17% to about 20%, about 17% to about 19%, about 17% to about 18%, about 18% to about 30%, about 18% to about 29%, about 18% to about 28%, about 18% to about 27%, about 18% to about 26%, about 18% to about 25%, about 18% to about 24%, about 18% to about 23%, about 18% to about 22%, about 18% to about 21%, about 18% to about 20%, about 18% to about 19%, about 19% to about 30%, about 19% to about 29%, about 19% to about 28%, about 19% to about 27%, about 19% to about 26%, about 19% to about 25%, about 19% to about 24%, about 19% to about 23%, about 19% to about 22%, about 19% to about 21%, about 19% to about 20%, about 20% to about 30%, about 20% to about 29%, about 20% to about 28%, about 20% to about 27%, about 20% to about 26%, about 20% to about 25%, about 20% to about 24%, about 20% to about 23%, about 20% to about 22%, about 20% to about 21%, about 21% to about 30%, about 21% to about 29%, about 21% to about 28%, about 21% to about 27%, about 21% to about 26%, about 21% to about 25%, about 21% to about 24%, about 21% to about 23%, about 21% to about 22%, about 22% to about 30%, about 22% to about 29%, about 22% to about 28%, about 22% to about 27%, about 22% to about 26%, about 22% to about 25%, about 22% to about 24%, about 22% to about 23%, about 23% to about 30%, about 23% to about 29%, about 23% to about 28%, about 23% to about 27%, about 23% to about 26%, about 23% to about 25%, about 23% to about 24%, about 24% to about 30%, about 24% to about 29%, about 24% to about 28%, about 24% to about 27%, about 24% to about 26%, about 24% to about 25%, about 25% to about 30%, about 25% to about 29%, about 25% to about 28%, about 25% to about 27%, about 25% to about 26%, about 26% to about 30%, about 26% to about 29%, about 26% to about 28%, about 26% to about 27%, about 27% to about 30%, about 27% to about 29%, about 27% to about 28%, about 28% to about 30%, about 28% to about 29%, or about 29% to about 30% by weight of the first cell mass (e.g., dry weight of the cell mass).

In some embodiments, the hydration mixture comprises at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least, 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95% by weight of the first cell mass (e.g., dry weight of the first cell mass).

In some embodiments, the water content of the first cell mass is adjusted to about 75% to about 95% (e.g., about 75% to about 90%, about 75% to about 85%, about 75% to about 80%, about 80% to about 95%, about 80% to about 90%, about 80% to about 85%, about 85% to about 95%, about 85% to about 90%, or about 90% to about 95%) by weight of water. In some embodiments, the water content of the first cell mass is at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% by weight of water. In some embodiments, the hydrated first cell mass comprises about 85% by weight of water content.

### 4.2.1.3 Flavoring agent

In some embodiments, the hydration mixture comprises one or more flavoring agents. Non-limiting examples of the one or more flavoring agents include: chicken white meat flavor, chicken breast flavor, salt, seasoning, herbs, and flavor enhancers.

In some embodiments, the hydration mixture comprises about 1.5% to about 4.5% (e.g., about 1.5% to about 4.0%, about 1.5% to about 3.5%, about 1.5% to about 2.5%, about 1.5% to about 2.0%, about 2.0% to about 4.5%, about 2.0% to about 4.0%, about 2.0% to about 3.5%, about 2.0% to about 3.0%, about 2.0% to about 2.5%, about 2.5% to about 4.5%, about 2.5% to about 4.0%, about 2.5% to about 3.5%, about 2.5% to about 3.0%, about 3.0% to about 4.5%, about 3.0% to about 4.0%, about 3.0% to about 3.5%, about 3.5% to about 4.5%, about 3.5% to about 4.0%, or about 4.0% to about 4.5%) by weight of a first flavoring agent (e.g., a salt).

In some embodiments, the hydration mixture comprises at least 1.5%, at least 1.6%, at least 1.7%, at least 1.8%, at least 1.9%, at least 2.0%, at least 2.1%, at least 2.2%, at least 2.3%, at least 2.4%, at least 2.5%, at least 2.6%, at least 2.7%, at least 2.8%, at least 2.9%, at least 3.0%, at least 3.1%, at least 3.2%, at least 3.3%, at least3.4%, at least 3.5%, at least 3.6%, at least 3.7%, at least 3.8%, at least 3.9%, at least 4.0%, at least 4.1%, at least 4.2%, at least 4.3%, at least 4.4%, at least 4.5%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, or at least 25% by weight of the first flavoring agent (e.g., a salt).

In some embodiments, the hydration mixture comprises about 3.5% to about 10.5% (e.g., 3.5% to about 9.5%, about 3.5% to about 8.5%, about 3.5% to about 7.5%, about 3.5% to about 6.5%, about 3.5% to about 5.5%, about 3.5% to about 4.5%, about 4.5% to about 10.5%, about 4.5% to about 9.5%, about 4.5% to about 8.5%, about 4.5% to about 7.5%, about 4.5% to about 6.5%, about 4.5% to about 5.5%, about 5.5% to about 10.5%, about 5.5% to about 9.5%, about 5.5% to about 8.5%, about 5.5% to about 7.5%, about 5.5% to about 6.5%, about 6.5% to about 10.5%, about 6.5% to about 9.5%, about 6.5% to about 8.5%, about 6.5% to about 7.5%, about 7.5% to about 10.5%, about 7.5% to about 9.5%, about 7.5% to about 8.5%, about 8.5% to about 10.5%, about 8.5% to about 9.5%, about 9.5% to about 10.5%) by weight of a second flavoring agent.

In some embodiments, the hydration mixture comprises at least 3.5%, at least 4.0%, at least 4.5%, at least 5.0%, at least 5.5%, at least 6.0%, at least 6.5%, at least 7.0%, at least 7.5%, at least 8.0%, at least 8.5%, at least 9.0%, at least 9.5%, at least 10.0%, at least 10.5%, at least 11%, at least 12%, at least 13%, at least 14%, at least 15%, at least 16%, at least 17%, at least 18%, at least 19%, at least 20%, at least 21%, at least 22%, at least 23%, at least 24%, or at least 25% by weight of a second flavoring agent.

### 4.2.2. Textured protein

In some embodiments, the cell based meat composition suitable for consumption comprises a textured protein. In some embodiments, a textured protein comprises a first textured protein and optionally a second textured protein. In some embodiments, a textured protein comprises a first textured protein. In some embodiments, a textured protein comprises a first textured protein and a second textured protein.

Suitable textured proteins (e.g., a first textured protein and/or a second textured protein) and suitable amounts of such textured proteins can be identified by titrating different textured proteins against the properties (e.g., without limitation, flavor, color, texture, size, shape, hydration capacity, off notes, hydration, and sensory profile) of the cell based meat composition to see which textured protein(s) produce the desired result.

In some embodiments, the cell based meat composition suitable for consumption comprises about 11% to about 33% (e.g., about 11% to about 32%, about 11% to about 31%, about 11% to about 30%, about 11% to about 29%, about 11% to about 28%, about 11% to about 27%, about 11% to about 26%, about 11% to about 25%, about 11% to about 24%, about 11% to about 23%, about 11% to about 22%, about 11% to about 21%, about 11% to about 20%, about 11% to about 19%, about 11% to about 18%, about 11% to about 17%, about 11% to about 16%, about 11% to about 15%, about 11% to about 14%, about 11% to about 13%, about 11% to about 12%, about 12% to about 33%, about 12% to about 32%, about 12% to about 31%, about 12% to about 30%, about 12% to about 29%, about 12% to about 28%, about 12% to about 27%, about 12% to about 26%, about 12% to about 25%, about 12% to about 24%, about 12% to about 23%, about 12% to about 22%, about 12% to about 21%, about 12% to about 20%, about 12% to about 19%, about 12% to about 18%, about 12% to about 17%, about 12% to about 16%, about 12% to about 15%, about 12% to about 14%, about 12% to about 13%, about 13% to about 33%, about 13% to about 32%, about 13% to about 31%, about 13% to about 30%, about 13% to about 29%, about 13% to about 28%, about 13% to about 27%, about 13% to about 26%, about 13% to about 25%, about 13% to about 24%, about 13% to about 23%, about 13% to about 22%, about 13% to about 21%, about 13% to about 20%, about 13% to about 19%, about 13% to about 18%, about 13% to about 17%, about 13% to about 16%, about 13% to about 15%, about 13% to about 14%, about 14% to about 33%, about 14% to about 32%, about 14% to about 31%, about 14% to about 30%, about 14% to about 29%, about 14% to about 28%, about 14% to about 27%, about 14% to about 26%, about 14% to about 25%, about 14% to about 24%, about 14% to about 23%, about 14% to about 22%, about 14% to about 21%, about 14% to about 20%, about 14% to about 19%, about 14% to about 18%, about 14% to about 17%, about 14% to about 16%, about 14% to about 15%, about 15% to about 33%, about 15% to about 32%, about 15% to about 31%, about 15% to about 30%, about 15% to about 29%, about 15% to about 28%, about 15% to about 27%, about 15% to about 26%, about 15% to about 25%, about 15% to about 24%, about 15% to about 23%, about 15% to about 22%, about 15% to about 21%, about 15% to about 20%, about 15% to about 19%, about 15% to about 18%, about 15% to about 17%, about 15% to about 16%, about 16% to about 33%, about 16% to about 32%, about 16% to about 31%, about 16% to about 30%, about 16% to about 29%, about 16% to about 28%, about 16% to about 27%, about 16% to about 26%, about 16% to about 25%, about 16% to about 24%, about 16% to about 23%, about 16% to about 22%, about 16% to about 21%, about 16% to about 20%, about 16% to about 19%, about 16% to about 18%, about 16% to about 17%, about 17% to about 33%, about 17% to about 32%, about 17% to about 31%, about 17% to about 30%, about 17% to about 29%, about 17% to about 28%, about 17% to about 27%, about 17% to about 26%, about 17% to about 25%, about 17% to about 24%, about 17% to about 23%, about 17% to about 22%, about 17% to about 21%, about 17% to about 20%, about 17% to about 19%, about 17% to about 18%, about 18% to about 33%, about 18% to about 32%, about 18% to about 31%, about 18% to about 30%, about 18% to about 29%, about 18% to about 28%, about 18% to about 27%, about 18% to about 26%, about 18% to about 25%, about 18% to about 24%, about 18% to about 23%, about 18% to about 22%, about 18% to about 21%, about 18% to about 20%, about 18% to about 19%, about 19% to about 33%, about 19% to about 32%, about 19% to about 31%, about 19% to about 30%, about 19% to about 29%, about 19% to about 28%, about 19% to about 27%, about 19% to about 26%, about 19% to about 25%, about 19% to about 24%, about 19% to about 23%, about 19% to about 22%, about 19% to about 21%, about 19% to about 20%, about 20% to about 33%, about 20% to about 32%, about 20% to about 31%, about 20% to about 30%, about 20% to about 29%, about 20% to about 28%, about 20% to about 27%, about 20% to about 26%, about 20% to about 25%, about 20% to about 24%, about 20% to about 23%, about 20% to about 22%, about 20% to about 21%, about 21% to about 33%, about 21% to about 32%, about 21% to about 31%, about 21% to about 30%, about 21% to about 29%, about 21% to about 28%, about 21% to about 27%, about 21% to about 26%, about 21% to about 25%, about 21% to about 24%, about 21% to about 23%, about 21% to about 22%, about 22% to about 33%, about 22% to about 32%, about 22% to about 31%, about 22% to about 30%, about 22% to about 29%, about 22% to about 28%, about 22% to about 27%, about 22% to about 26%, about 22% to about 25%, about 22% to about 24%, about 22% to about 23%, about 23% to about 33%, about 23% to about 32%, about 23% to about 31%, about 23% to about 30%, about 23% to about 29%, about 23% to about 28%, about 23% to about 27%, about 23% to about 26%, about 23% to about 25%, about 23% to about 24%, about 24% to about 33%, about 24% to about 32%, about 24% to about 31%, about 24% to about 30%, about 24% to about 29%, about 24% to about 28%, about 24% to about 27%, about 24% to about 26%, about 24% to about 25%, about 25% to about 33%, about 25% to about 32%, about 25% to about 31%, about 25% to about 30%, about 25% to about 29%, about 25% to about 28%, about 25% to about 27%, about 25% to about 26%, about 26% to about 33%, about 26% to about 32%, about 26% to about 31%, about 26% to about 30%, about 26% to about 29%, about 26% to about 28%, about 26% to about 27%, about 27% to about 33%, about 27% to about 32%, about 27% to about 31%, about 27% to about 30%, about 27% to about 29%, about 27% to about 28%, about 28% to about 33%, about 28% to about 32%, about 28% to about 31%, about 28% to about 30%, about 28% to about 29%, about 29% to about 33%, about 29% to about 32%, about 29% to about 31%, about 29% to about 30%, about 30% to about 33%, about 30% to about 32%, about 30% to about 31%, about 31% to about 33%, about 31% to about 32%, or about 31% to about 32%) by weight of a textured protein (e.g., a first textured, a second textured protein, or both).

In some embodiments, the cell based meat composition suitable for consumption comprises at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44% at least 45%, at least 46%, at least 47%, at least 48%, at least 49%, at least 50%, at least 55%, at least 60%, at least 65%, at least 70%, at least 75%, at least 80%, at least 85 %, at least 90%, or at least 95% by weight of a textured protein (e.g., a first textured, a second textured protein, or both).

In some embodiments, the first textured protein, the second textured protein, or both are selected based on factors including but not limited to: flavor, color, hydration capacity, and sensory profile. In some embodiments, the first textured protein, the second textured protein, or both are textured soy proteins (TSP). In some embodiments, the first textured protein, second textured protein, or both, are any textured protein that contributes, at least in part, to a desired water activity or desired texture profile of the cell based meat composition. In some embodiments, the first textured protein, second textured protein, or both, include pea, lentil, mung bean, or a combination thereof. In some embodiments, the first textured protein, second textured protein, or both, are not pea, not lentil, not mung bean, nor any combination thereof.

In some embodiments, the first textured protein, the second textured protein, or both are selected from a TSP having the following characteristics: a granule of about a 2 mm in diameter; a cube of about 6 mm to about 20 mm by about 6 mm to about 20 mm; a flake of about 2 mm in diameter by about 10 to about 70 mm in length; and a chunk of about 15 mm to about 25 mm in diameter by about 30 mm to about 70 mm in length, or a combination thereof. In some embodiments, the first textured protein, the second textured protein, or both are selected from a TSP having a granule of about 0.1 mm, about 0.2 mm, about 0.3 mm, about 0.4 mm, about 0.5 mm, about 0.6 mm, about 0.7 mm, about 0.8 mm, about 0.9 mm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, or about 2.0 mm.

In some embodiments, the first textured protein, the second textured protein, or both, comprises a water content to a textured protein ratio of about 2.60 to about 2.85 (e.g., about 2.6 to about 2.8, about 2.6 to about 2.75, about 2.6 to about 2.7, about 2.6 to about 2.65, about 2.65 to about 2.85, about 2.65 to about 2.8, about 2.65 to about 2.75, about 2.65 to about 2.7, about 2.7 to about 2.85, about 2.7 to about 2.8, about 2.7 to about 2.75, about 2.75 to about 2.85, about 2.75 to about 2.8, or about 2.8 to about 2.85).

In some embodiments, the first textured protein comprises one or more of the following characteristics: uncolored, neutral flavor, and a granule size of about 2 mm to about 6 mm (e.g., about 2 mm to about 5 mm, about 2 mm to about 4 mm, about 2 mm to about 3 mm, about 3 mm to about 6 mm, about 3 mm to about 5 mm, about 3 mm to about 4 mm, about 4 mm to about 6 mm, about 4 mm to about 5 mm or about 5 mm to about 6 mm).

In some embodiments, the second textured protein comprises one or more of the following characteristics: uncolored, neutral flavor, and a flake size of about 2 mm to about 6 mm (e.g., about 2 mm to about 5 mm, about 2 mm to about 4 mm, about 2 mm to about 3 mm, about 3 mm to about 6 mm, about 3 mm to about 5 mm, about 3 mm to about 4 mm, about 4 mm to about 6 mm, about 4 mm to about 5 mm or about 5 mm to about 6 mm)..

### 4.2.3. Dry Blend

In some embodiments, the cell based meat composition includes a dry blend. In some embodiments, a dry blend can be used to add flavor, texture, or color to the cell based meat composition. In some embodiments, the dry blend can be used to add a third cell mass to the cell based meat composition.

In some embodiments, the cell based meat composition suitable for consumption comprises about 1% to about 12% (e.g., about 1% to about 11%, about 1% to about 10%, about 1% to about 9%, about 1% to about 8%, about 1% to about 7%, about 1% to about 6%, about 1% to about 5%, about 1% to about 4%, about 1% to about 3%, about 1% to about 2%, about 2% to about 12%, about 2% to about 11%, about 2% to about 10%, about 2% to about 9%, about 2% to about 8%, about 2% to about 7%, about 2% to about 6%, about 2% to about 5%, about 2% to about 4%, about 2% to about 3%, about 3% to about 12%, about 3% to about 11%, about 3% to about 10%, about 3% to about 9%, about 3% to about 8%, about 3% to about 7%, about 3% to about 6%, about 3% to about 5%, about 3% to about 4%, about 4% to about 12%, about 4% to about 11%, about 4% to about 10%, about 4% to about 9%, about 4% to about 8%, about 4% to about 7%, about 4% to about 6%, about 4% to about 5%, about 5% to about 12%, about 5% to about 11%, about 5% to about 10%, about 5% to about 9%, about 5% to about 8%, about 5% to about 7%, about 5% to about 6%, about 6% to about 12%, about 6% to about 11%, about 6% to about 10%, about 6% to about 9%, about 6% to about 8%, about 6% to about 7%, about 7% to about 12%, about 7% to about 11%, about 7% to about 10%, about 7% to about 9%, about 7% to about 8%, about 8% to about 12%, about 8% to about 11%, about 8% to about 10%, about 8% to about 9%, about 9% to about 12%, about 9% to about 11%, about 9% to about 10%, about 10% to about 12%, about 10% to about 11%, or about 11% to about 12%) by weight of a dry blend.

In some embodiments, the cell based meat composition suitable for consumption comprises at least 0.001%, at least 0.01%, at least 0.05%, at least 0.1%, at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 17%, at least 18%, at least 19%, at least 20%, at least 21%, at least 22%, at least 23%, at least 24%, at least 25% by weight of a dry blend.

In some embodiments, the dry blend comprises about 50% to about 70% by weight of a third textured protein; and about 30% to about 50% by weight of a flavoring agent (e.g., any of the flavoring agents described herein).

In some embodiments, the dry blend comprises about 50% to about 70% (e.g., about 50% to about 65%, about 50% to about 60%, about 50% to about 55%, about 55% to about 70%, about 55% to about 65%, about 55% to about 60%, about 60% to about 70%, about 60% to about 65%, or about 65% to about 70%) by weight of a third textured protein.

In some embodiments, the dry blend comprises at least 25%, at least 30%, at least 35%, at least 40%, at least 45%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69% or at least 70%, at least 75%, at least 80%, at least 85 %, at least 90%, or at least 95% by weight of the third textured protein.

In some embodiments, the third textured protein is selected based on factors, including but not limited to: flavor, color, hydration capacity, and sensory profile. In some embodiments, the third textured protein comprises a textured soy protein (TSP).

In some embodiments, the third textured protein includes any of the textured proteins described herein (see, e.g., Section 4.2.2).

In some embodiments, the third textured protein is hydrated. In some embodiments, the third textured protein is not hydrated.

In some embodiments, the dry blend comprises about 30% to about 50% (e.g., about 30% to about 45%, about 30% to about 40%, about 30% to about 35%, about 35% to about 50%, about 35% to about 45%, about 35% to about 40%, about 40% to about 50%, about 40% to about 45%, or about 45% to about 50%) by weight of a flavoring agent (e.g., any of the flavoring agents described herein.

In some embodiments, the dry blend comprises at least 30%, at least 31%, at least 32%, at least 33%, at least 34%, at least 35%, at least 36%, at least 37%, at least 38%, at least 39%, at least 40%, at least 41%, at least 42%, at least 43%, at least 45%, at least 46%, at least 47%, at least 48%, at least 49% or at least 50% by weight of the flavoring agent (e.g., the second flavoring agent).

In some embodiments, the dry blend comprises about 58% by weight of a third textured protein and about 42% by weight of a flavoring agent (e.g., a second flavoring agent).

In some embodiments, the dry blend includes a third cell mass. In some embodiments, the third cell mass comprises a population of cells harvested from suspension culture. In some embodiments, the population of cells is harvested from *in vitro* in suspension culture. In some embodiments, the population of cells is separated from the components of the suspension culture. Separation can be achieved using filtration, centrifugation, gravity, spray drying, or a combination thereof. Once separated from the components of the suspension culture (e.g., the cell culture medium), the population of cells may comprise a dry cell pellet. Non-limiting examples of solution include: water, saline solution, serum-free cell culture medium, and conditioned (filtered) medium from the suspension culture.

In some embodiments, the third cell mass (e.g., the hydrated third cell mass) comprises about 1.0 ×10⁶ to about 1× 10¹¹ (e.g., about 1.0 ×10⁶ to about 1× 10¹⁰, about 1.0 ×10⁶ to about 1× 10⁹, about 1.0 ×10⁶ to about 1× 10⁸, about 1.0 ×10⁶ to about 1× 10⁷, about 1.0 ×10⁷ to about 1× 10¹¹, about 1.0 ×10⁷ to about 1× 10¹⁰, about 1.0 ×10⁷ to about 1× 10⁹, about 1.0 ×10⁷ to about 1× 10⁸, about 1.0 ×10⁸ to about 1× 10¹¹, about 1.0 ×10⁸ to about 1× 10¹⁰, about 1.0 ×10⁸ to about 1× 10⁹, about 1.0 ×10⁹ to about 1× 10¹¹, about 1.0 ×10⁹ to about 1× 10¹⁰, or about 1.0 ×10¹⁰ to about 1× 10¹¹) cells.

In some embodiments, the third cell mass (e.g., the hydrated third cell mass) comprises a packed cell volume (PCV) of at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95%.

In some embodiments, the dry weight of the third cell mass (e.g., the population of cells harvested from the suspension culture) comprises at least about 0.001 grams (g), at least about 0.005 g, at least about 0.01 g, at least about 0.05 g, at least about 0.1 g, at least about 0.5 g, at least about 1.0 g, at least about 2.0 g, at least about 3.0 g, at least about 4.0 g, at least about 5.0 g, at least about 6.0 g, at least about 7.0 g, at least about 8.0 g, at least about 9.0 g, at least about 10.0 g, at least about 11 g, at least about 12 g, at least about 13 g, at least about 14 g, at least about 15 g, at least about 16 g, at least about 17 g, at least about 18 g, at least about 19 g, at least about 20 g, at least about 21 g, at least about 22 g, at least about 23 g, at least about 24 g, at least about 25 g, at least about 26 g, at least about 27 g, at least about 28 g, at least about 29 g, at least about 30 g, at least about 31 g, at least about 32 g, at least about 33 g, at least about 34 g, at least about 35 g, at least about 36 g, at least about 37 g, at least about 38 g, at least about 39 g, at least about 40 g, at least about 41 g, at least about 42 g, at least about 43 g, at least about 44 g, at least about 45 g, at least about 46 g, at least about 47 g, at least about 48 g, at least about 49 g, or at least about 50 g.

In some embodiments, the dry blend comprises about 50% to about 70% (e.g., about 50% to about 65%, about 50% to about 60%, about 50% to about 55%, about 55% to about 70%, about 55% to about 65%, about 55% to about 60%, about 60% to about 70%, about 60% to about 65%, or about 65% to about 70%) by weight of a third cell mass (e.g., a third hydrated cell mass).

In some embodiments, the dry blend comprises at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95% by weight of a third cell mass (e.g., a third hydrated cell mass).

In some embodiments, the water content of the third cell mass is adjusted to about 75% to about 95% (e.g., about 75% to about 90%, about 75% to about 85%, about 75% to about 80%, about 80% to about 95%, about 80% to about 90%, about 80% to about 85%, about 85% to about 95%, about 85% to about 90%, or about 90% to about 95%) by weight of water. In some embodiments, the water content of the third cell mass is at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% by weight of water. In some embodiments, the hydrated third cell mass comprises about 85% by weight of water.

### 4.2.4. Emulsion Mixture

In some embodiments, the cell based meat composition suitable for consumption comprises an emulsion mixture. Without wishing to be bound by theory, the emulsion mixture acts, at least in part, to form a gel for binding and shape retention for the cell based meat composition. In some settings , emulsion mixtures require heat to be able to impart these qualities (i.e., binding and shape retention) on the mixture (i.e., the cell based meat composition).

Examples of suitable emulsion mixtures include but are not limited to water-in-oil emulsions, oil-in-water emulsions, oil-in-water-in-oil double emulsions, water-in-oil-in-water double emulsions, and Pickering emulsions. The presence of an emulsion mixture in any of the cell based meat composition described herein can be determined semi-quantitatively by a combination of selective staining and microscopic observation using brightfield, fluorescence, or phase contrast microscopy. In such cases, observation using transmitted or reflected light may be required to distinguish the emulsion droplets within the cell based meat composition.

In some embodiments, the emulsion mixture includes droplets of various sizes. In some embodiments, the emulsion mixture in the cell based meat compositions described herein are polydisperse emulsions (i.e., emulsions comprising droplets with a broad distribution of droplet sizes). In some embodiments, the emulsion mixtures are monodisperse (i.e., emulsions comprising droplets with a narrow distribution of droplet sizes). In some embodiments, the emulsion mixtures are microemulsions (i.e., thermodynamic stable systems of dispersed droplets in continuous phase). In some embodiments, the emulsion mixtures are nanoemulsions (i.e., kinetically stable dispersions of one liquid in another immiscible liquid having droplet sizes ranging from 1 to 100 nm).

In some embodiments, the emulsion mixture comprises an average droplet size of less than about 1000 nm, about 900 nm, about 800 nm, about 700 nm, about 600 nm, about 500 nm, about 400 nm, about 300 nm, about 200 nm, about 100 nm, about 75 nm, about 50 nm, about 25 nm, about 15 nm, about 10 nm, or about 5 nm.

In some embodiments, the emulsion mixture comprises an average droplet size of between about 50 nm to about 1000 nm (e.g., about 50 nm to about 900 nm, about 50 nm to about 800 nm, about 50 nm to about 700 nm, about 50 nm to about 600 nm, about 50 nm to about 500 nm, about 50 nm to about 400 nm, about 50 nm to about 300 nm, about 50 nm to about 200 nm, about 50 nm to about 100 nm, about 100 nm to about 1000 nm, about 100 nm to about 900 nm, about 100 nm to about 800 nm, about 100 nm to about 700 nm, about 100 nm to about 600 nm, about 100 nm to about 500 nm, about 100 nm to about 400 nm, about 100 nm to about 300 nm, about 100 nm to about 200 nm, about 200 nm to about 1000 nm, about 200 nm to about 900 nm, about 200 nm to about 800 nm, about 200 nm to about 700 nm, about 200 nm to about 600 nm, about 200 nm to about 500 nm, about 200 nm to about 400 nm, about 200 nm to about 300 nm, about 300 nm to about 1000 nm, about 300 nm to about 900 nm, about 300 nm to about 800 nm, about 300 nm to about 700 nm, about 300 nm to about 600 nm, about 300 nm to about 500 nm, about 300 nm to about 400 nm, about 400 nm to about 1000 nm, about 400 nm to about 900 nm, about 400 nm to about 800 nm, about 400 nm to about 700 nm, about 400 nm to about 600 nm, about 400 nm to about 500 nm, about 500 nm to about 1000 nm, about 500 nm to about 900 nm, about 500 nm to about 800 nm, about 500 nm to about 700 nm, about 500 nm to about 600 nm, about 600 nm to about 1000 nm, about 600 nm to about 900 nm, about 600 nm to about 800 nm, about 600 nm to about 700 nm, about 700 nm to about 1000 nm, about 700 nm to about 900 nm, about 700 nm to about 800 nm, about 800 nm to about 1000 nm, about 800 nm to about 900 nm, or about 900 nm to about 1000 nm).

In some embodiments, the emulsion mixture comprises an average droplet size of between about 1 µm and about 10 µm, about 2 µm and about 9 µm, about 3 µm to about 8 µm, about 4 µm to about 7 µm, or about 5 µm to about 6 µm.

The size of droplets of the emulsion mixture can be determined by methods known in the art including but not limited to light scattering, microscopy, and spectroscopy.

In some embodiments, the emulsion mixture remains stable against droplet coalescence and gravitational creaming for at least 7 days, at least 10 days, or at least 14 days when stored at 4°C.

In some embodiments, the internal temperature of the emulsion mixture remains below at least 35°C (e.g., at least below 30°C, at least below 25°C, at least below 20°C, at least below 15°C, at least below 10°C, or at least below 5°C).

In some embodiments, the emulsion mixture comprises an oil, a hydrocolloid, and a hydrated second cell mass.

In some embodiments, the cell based meat composition suitable for consumption comprises about 5% to about 70% (e.g., about 5% to about 65%, about 5% to about 60%, about 5% to about 55%, about 5% to about 50%, about 5% to about 45%, about 5% to about 45%, about 5% to about 40%, about 5% to about 35%, about 5% to about 30%, about 5% to about 25%, about 5% to about 20%, about 5% to about 15%, about 5% to about 10%, about 10% to about 70%, about 10% to about 65%, about 10% to about 60%, about 10% to about 55%, about 10% to about 50%, about 10% to about 45%, about 10% to about 40%, about 10% to about 35%, about 10% to about 30%, about 10% to about 25%, about 10% to about 20%, about 10% to about 15%, about 15% to about 70%, about 15% to about 65%, about 15% to about 60%, about 15% to about 55%, about 15% to about 50%, about 15% to about 45%, about 15% to about 40%, about 15% to about 35%, about 15% to about 30%, about 15% to about 25%, about 15% to about 20%, about 20% to about 70%, about 20% to about 65%, about 20% to about 60%, about 20% to about 55%, about 20% to about 50%, about 20% to about 45%, about 20% to about 40%, about 20% to about 35%, about 20% to about 30%, about 20% to about 25%, about 25% to about 70%, about 25% to about 65%, about 25% to about 60%, about 25% to about 55%, about 25% to about 50%, about 25% to about 45%, about 25% to about 40%, about 25% to about 35%, about 25% to about 30%, about 30% to about 70%, about 30% to about 65%, about 30% to about 60%, about 30% to about 55%, about 30% to about 50%, about 30% to about 45%, about 30% to about 40%, about 30% to about 35%, about 35% to about 70%, about 35% to about 65%, about 35% to about 60%, about 35% to about 55%, about 35% to about 50%, about 35% to about 45%, about 35% to about 40%, about 40% to about 70%, about 40% to about 65%, about 40% to about 60%, about 40% to about 55%, about 40% to about 50%, about 40% to about 45%, about 45% to about 70%, about 45% to about 65%, about 45% to about 60%, about 45% to about 55%, about 45% to about 50%, about 50% to about 70%, about 50% to about 65%, about 50% to about 60%, about 50% to about 55%, about 55% to about 70%, about 55% to about 65%, about 55% to about 60%, about 60% to about 70%, about 60% to about 65%, or about 65% to about 70%) by weight of an emulsion mixture (e.g., an emulsion mixture comprising an oil, a hydrocolloid and a hydrated second cell mass).

In some embodiments, the cell based meat composition suitable for consumption comprises at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 45%, at least 46%, at least 47%, at least 48%, at least 49%, at least 50%, at least 55%, at least 60%, at least 65%, at least 70%, at least 75%, at least 80%, at least 85 %, at least 90%, or at least 95% by weight of an emulsion mixture (e.g., an emulsion mixture comprising an oil, a hydrocolloid and a hydrated second cell mass).

In some embodiments, the emulsion mixtures comprises: about 8% to about 24% by weight of oil; about 2.5% to about 7.5% by weight of the hydrocolloid; and about 55% to about 95% by weight of the second cell mass.

In some embodiments, the emulsion mixture comprises a viscosity of about 1 cps to about 10,000 cps.

### 4.2.4.1 Oil

In some embodiments, the emulsion mixture comprises an oil.

In some embodiments, the emulsion mixture comprises about 8% to about 24% (e.g., about 8% to about 23%, about 8% to about 22%, about 8% to about 21%, about 8% to about 20%, about 8% to about 19%, about 8% to about 18%, about 8% to about 17%, about 8% to about 16%, about 8% to about 15%, about 8% to about 14%, about 8% to about 13%, about 8% to about 12%, about 8% to about 11%, about 8% to about 10%, about 8% to about 9%, about 9% to about 24%, about 9% to about 23%, about 9% to about 22%, about 9% to about 21%, about 9% to about 20%, about 9% to about 19%, about 9% to about 18%, about 9% to about 17%, about 9% to about 16%, about 9% to about 15%, about 9% to about 14%, about 9% to about 13%, about 9% to about 12%, about 9% to about 11%, about 9% to about 10%, about 10% to about 24%, about 10% to about 23%, about 10% to about 22%, about 10% to about 21%, about 10% to about 20%, about 10% to about 19%, about 10% to about 18%, about 10% to about 17%, about 10% to about 16%, about 10% to about 15%, about 10% to about 14%, about 10% to about 13%, about 10% to about 12%, about 10% to about 11%, about 11% to about 24%, about 11% to about 23%, about 11% to about 22%, about 11% to about 21%, about 11% to about 20%, about 11% to about 19%, about 11% to about 18%, about 11% to about 17%, about 11% to about 16%, about 11% to about 15%, about 11% to about 14%, about 11% to about 13%, about 11% to about 12%, about 12% to about 24%, about 12% to about 23%, about 12% to about 22%, about 12% to about 21%, about 12% to about 20%, about 12% to about 19%, about 12% to about 18%, about 12% to about 17%, about 12% to about 16%, about 12% to about 15%, about 12% to about 14%, about 12% to about 13%, about 13% to about 24%, about 13% to about 23%, about 13% to about 22%, about 13% to about 21%, about 13% to about 20%, about 13% to about 19%, about 13% to about 18%, about 13% to about 17%, about 13% to about 16%, about 13% to about 15%, about 13% to about 14%, about 14% to about 24%, about 14% to about 23%, about 14% to about 22%, about 14% to about 21%, about 14% to about 20%, about 14% to about 19%, about 14% to about 18%, about 14% to about 17%, about 14% to about 16%, about 14% to about 15%, about 15% to about 24%, about 15% to about 23%, about 15% to about 22%, about 15% to about 21%, about 15% to about 20%, about 15% to about 19%, about 15% to about 18%, about 15% to about 17%, about 15% to about 16%, about 16% to about 24%, about 16% to about 23%, about 16% to about 22%, about 16% to about 21%, about 16% to about 20%, about 16% to about 19%, about 16% to about 18%, about 16% to about 17%, about 17% to about 24%, about 17% to about 23%, about 17% to about 22%, about 17% to about 21%, about 17% to about 20%, about 17% to about 19%, about 17% to about 18%, about 18% to about 24%, about 18% to about 23%, about 18% to about 22%, about 18% to about 21%, about 18% to about 20%, about 18% to about 19%, about, about 19% to about 24%, about 19% to about 23%, about 19% to about 22%, about 19% to about 21%, about 19% to about 20%, about 20% to about 24%, about 20% to about 23%, about 20% to about 22%, about 20% to about 21%, about 21% to about 24%, about 21% to about 23%, about 21% to about 22%, about 22% to about 24%, about 22% to about 23%, or about 23% to about 24%) by weight of oil.

In some embodiments, the emulsion mixture comprises at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24% at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95% by weight of oil. by weight of oil. In some embodiments, the emulsion mixture comprises at least about 8% by weight of oil.

In some embodiments, the oil is selected from soybean oil, coconut oil, mango oil, sunflower oil, cottonseed oil, safflower oil, rice bran oil, cocoa butter, palm kernel oil, palm fruit oil, palm oil, rapeseed oil, canola oil, com oil, sesame oil, walnut oil, almond oil, flaxseed, jojoba oil, castor, grapeseed oil, peanut oil, olive oil, borage oil, algal oil, fungal oil, black currant oil, babassu oil, shea butter, mango butter, wheat germ oil, blackcurrant oil, sea-buckhorn oil, macadamia oil, and saw palmetto oil. In some embodiments, the oil is soybean oil.

In another embodiments, the emulsion mixture comprises a lipid. In some embodiments, the emulsion mixture comprises at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, at least 17%, at least 18%, at least 19%, at least 20%, at least 21%, at least 22%, at least 23%, at least 24% at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95% by weight of a lipid.

### 4.2.4.2 Hydrocolloid

In some embodiments, an emulsion mixture comprises a hydrocolloid. In some embodiments, an emulsion mixture comprises methylcellulose. Without wishing to be bound by theory, the hydrocolloid acts, at least in part, to form a gel for binding and shape retention for the cell based meat composition and is not used for creating crosslinks between the cell masses and textured proteins. Suitable hydrocolloids and suitable amounts of such hydrocolloids can be identified by titrating different binding agents against the cohesiveness, binding, and malleability of cell based meat compositions. A suitable assay for determining binding enabled by the hydrocolloid is disclosed in Example 3.

In some embodiments, the emulsion mixture comprises about 2.5% to about 7.5% (e.g., about 2.5% to about 7.25%, about 2.5% to about 7.0%, about 2.5% to about 6.75%, about 2.5% to about 6.5%, about 2.5% to about 6.25%, about 2.5% to about 6.0%, about 2.5% to about 5.75%, about 2.5% to about 5.5%, about 2.5% to about 5.25%, about 2.5% to about 5.0%, about 2.5% to about 4.75%, about 2.5% to about 4.5%, about 2.5% to about 4.25%, about 2.5% to about 4.0%, about 2.5% to about 3.75%, about 2.5% to about 3.5%, about 2.5% to about 3.25%, about 2.5% to about 3.0%, about 2.5% to about 2.75%, about 2.75% to about 7.5%, about 2.75% to about 7.5%, about 2.75% to about 7.25%, about 2.75% to about 7.0%, about 2.75% to about 6.75%, about 2.75% to about 6.5%, about 2.75% to about 6.25%, about 2.75% to about 6.0%, about 2.75% to about 5.75%, about 2.75% to about 5.5%, about 2.75% to about 5.25%, about 2.75% to about 5.0%, about 2.75% to about 4.75%, about 2.75% to about 4.5%, about 2.75% to about 4.25%, about 2.75% to about 4.0%, about 2.75% to about 3.75%, about 2.75% to about 3.5%, about 2.75% to about 3.25%, about 2.75% to about 3.0%, about 3.0% to about 7.5%, about 3.0% to about 7.25%, about 3.0% to about 7.0%, about 3.0% to about 6.75%, about 3.0% to about 6.5%, about 3.0% to about 6.25%, about 3.0% to about 6.0%, about 3.0% to about 5.75%, about 3.0% to about 5.5%, about 3.0% to about 5.25%, about 3.0% to about 5.0%, about 3.0% to about 4.75%, about 3.0% to about 4.5%, about 3.0% to about 4.25%, about 3.0% to about 4.0%, about 3.0% to about 3.75%, about 3.0% to about 3.5%, about 3.0% to about 3.25%, about 3.25% to about 7.5%, about 3.25% to about 7.25%, about 3.25% to about 7.0%, about 3.25% to about 6.75%, about 3.25% to about 6.5%, about 3.25% to about 6.25%, about 3.25% to about 6.0%, about 3.25% to about 5.75%, about 3.25% to about 5.5%, about 3.25% to about 5.25%, about 3.25% to about 5.0%, about 3.25% to about 4.75%, about 3.25% to about 4.5%, about 3.25% to about 4.25%, about 3.25% to about 4.0%, about 3.25% to about 3.75%, about 3.25% to about 3.5%, about 3.5% to about 7.5%, about 3.5% to about 7.25%, about 3.5% to about 7.0%, about 3.5% to about 6.75%, about 3.5% to about 6.5%, about 3.5% to about 6.25%, about 3.5% to about 6.0%, about 3.5% to about 5.75%, about 3.5% to about 5.5%, about 3.5% to about 5.25%, about 3.5% to about 5.0%, about 3.5% to about 4.75%, about 3.5% to about 4.5%, about 3.5% to about 4.25%, about 3.5% to about 4.0%, about 3.5% to about 3.75%, about 3.75% to about 7.5%, about 3.75% to about 7.25%, about 3.75% to about 7.0%, about 3.75% to about 6.75%, about 3.75% to about 6.5%, about 3.75% to about 6.25%, about 3.75% to about 6.0%, about 3.75% to about 5.75%, about 3.75% to about 5.5%, about 3.75% to about 5.25%, about 3.75% to about 5.0%, about 3.75% to about 4.75%, about 3.75% to about 4.5%, about 3.75% to about 4.25%, about 3.75% to about 4.0%, about 4.0% to about 7.5%, about 4.0% to about 7.25%, about 4.0% to about 7.0%, about 4.0% to about 6.75%, about 4.0% to about 6.5%, about 4.0% to about 6.25%, about 4.0% to about 6.0%, about 4.0% to about 5.75%, about 4.0% to about 5.5%, about 4.0% to about 5.25%, about 4.0% to about 5.0%, about 4.0% to about 4.75%, about 4.0% to about 4.5%, about 4.0% to about 4.25%, about 4.25% to about 7.5%, about 4.25% to about 7.25%, about 4.25% to about 7.0%, about 4.25% to about 6.75%, about 4.25% to about 6.5%, about 4.25% to about 6.25%, about 4.25% to about 6.0%, about 4.25% to about 5.75%, about 4.25% to about 5.5%, about 4.25% to about 5.25%, about 4.25% to about 5.0%, about 4.25% to about 4.75%, about 4.25% to about 4.5%, about 4.5% to about 7.5%, about 4.5% to about 7.25%, about 4.5% to about 7.0%, about 4.5% to about 6.75%, about 4.5% to about 6.5%, about 4.5% to about 6.25%, about 4.5% to about 6.0%, about 4.5% to about 5.75%, about 4.5% to about 5.5%, about 4.5% to about 5.25%, about 4.5% to about 5.0%, about 4.5% to about 4.75%, about 4.75% to about 7.5%, about 4.75% to about 7.25%, about 4.75% to about 7.0%, about 4.75% to about 6.75%, about 4.75% to about 6.5%, about 4.75% to about 6.25%, about 4.75% to about 6.0%, about 4.75% to about 5.75%, about 4.75% to about 5.5%, about 4.75% to about 5.25%, about 4.75% to about 5.0% about 5.0% to about 7.5%, about 5.0% to about 7.0%, about 5.0% to about 6.75%, about 5.0% to about 6.5%, about 5.0% to about 6.25%, about 5.0% to about 6.0%, about 5.0% to about 5.75%, about 5.0% to about 5.5%, about 5.25% to about 7.5%, about 5.25% to about 7.25%, about 5.25% to about 7.0%, about 5.25% to about 6.75%, about 5.25% to about 6.5%, about 5.25% to about 6.25%, about 5.25% to about 6.0%, about 5.25% to about 5.75%, about 5.25% to about 5.5%, about 5.5% to about 7.5%, about 5.5% to about 7.25%, about 5.5% to about 7.0%, about 5.5% to about 6.75%, about 5.5% to about 6.5%, about 5.5% to about 6.25%, about 5.5% to about 6.0%, about 5.5% to about 5.75%, about 5.75% to about 7.5%, about 5.5% to about 7.25%, about 5.75% to about 7.0%, about 5.75% to about 6.75%, about 5.75% to about 6.5%, about 5.75% to about 6.25%, about 5.75% to about 6.0%, about 6.0% to about 7.5%, about 6.0% to about 7.25%, about 6.0% to about 7.0%, about 6.0% to about 6.75%, about 6.0% to about 6.5%, about 6.0% to about 6.25%, about 6.25% to about 7.5%, about 6.25% to about 7.25%, about 6.25% to about 7.0%, about 6.25% to about 6.75%, about 6.25% to about 6.5%, about 6.5% to about 7.5%, about 6.5% to about 7.0%, about 6.5% to about 6.75%, about 6.75% to about 7.5%, about 6.75% to about 7.25%, about 6.75% to about 7.0%, about 7.0% to about 7.5%, about 7.0% to about 7.25%, or about 7.25% to about 7.5%) by weight of the hydrocolloid.

In some embodiments, the emulsion mixture comprises at least 0.01%, at least 0.05%, at least 0.1%, at least 0.5%, at least 1.0%, at least 1.5%, at least 2.0%, at least 2.5%, at least 3.0%, at least 3.5%, at least 4.0%, at least 4.5%, at least 5.0%, at least 5.5%, at least 6.0%, at least 6.5%, at least 7.0%, at least 7.5%, at least 8.0%, at least 8.5%, at least 9.0%, at least 9.5%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95% by weight of the hydrocolloid. In some embodiments, the emulsion mixture comprises at least 2.5% by weight of the hydrocolloid.

In some embodiments, the hydrocolloid is selected from methylcellulose, agar gel, gellan gum, konjac mannan, carrageenan, furcellaran, alginate, gum arabic, gum ghatti, gum tragacanth, karaya gum, guar gum, locust bean gum, tara gum, tamarind gum, inulin, arabinoxylans, b-glucans, xyloglucans, pectin, cellulose, curdlan, dextran, rhamsan gum, scleroglucan, welan gum, xanthan gum, gelatin, carboxymethylcellulose, hydroxypropyl cellulose, hydroxypropyl methyl cellulose, hydroxyethyl cellulose, propylene glycol alginate, hydroxypropyl guar, modified starches, and mixtures thereof. In some embodiments, the hydrocolloid is methylcellulose.

### 4.2.4.3 Second Cell Mass

In some embodiments, the emulsion mixture comprises a second cell mass. In some embodiments, the second cell mass comprises a population of cells harvested from suspension culture. In some embodiments, the population of cells is harvested from *in vitro* in suspension culture. In some embodiments, the population of cells is separated from the components of the suspension culture. Separation can be achieved using filtration, centrifugation, gravity, spray dry, or a combination thereof. Once separated from the components of the suspension culture (e.g., the cell culture medium), the population of cells may comprise a dry cell pellet or dry cell powder. The cell pellet or powder may be hydrated with a solution to produce the hydrated second cell mass. Non-limiting examples of a solution include: water, saline solution, serum-free cell culture medium, and conditioned (filtered) medium from the suspension culture.

In some embodiments, the second cell mass (e.g., the hydrated second cell mass) comprises about 1.0 ×10⁶ to about 1× 10¹¹ (e.g., about 1.0 ×10⁶ to about 1× 10¹⁰, about 1.0 ×10⁶ to about 1× 10⁹, about 1.0 ×10⁶ to about 1× 10⁸, about 1.0 ×10⁶ to about 1× 10⁷, about 1.0 ×10⁷ to about 1× 10¹¹, about 1.0 ×10⁷ to about 1× 10¹⁰, about 1.0 ×10⁷ to about 1× 10⁹, about 1.0 ×10⁷ to about 1× 10⁸, about 1.0 ×10⁸ to about 1× 10¹¹, about 1.0 ×10⁸ to about 1× 10¹⁰, about 1.0 ×10⁸ to about 1× 10⁹, about 1.0 ×10⁹ to about 1× 10¹¹, about 1.0 ×10⁹ to about 1× 10¹⁰, or about 1.0 ×10¹⁰ to about 1× 10¹¹) cells.

In some embodiments, the second cell mass (e.g., the hydrated second cell mass) comprises a packed cell volume (PCV) of at least 0.5%, at least 1%, at least 2%, at least 3%, at least 4%, at least 5%, at least 6%, at least 7%, at least 8%, at least 9%, at least 10%, at least 11%, at least 12%, at least 13%, at least 14%, at least 15%, least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79% at least 80%, at least 85 %, at least 90%, or at least 95%.

In some embodiments, the dry weight of the second cell mass (e.g., the population of cells harvested from the suspension culture) comprises at least about 0.001 grams (g), at least about 0.005 g, at least about 0.01 g, at least about 0.05 g, at least about 0.1 g, at least about 0.5 g, at least about 1.0 g, at least about 2.0 g, at least about 3.0 g, at least about 4.0 g, at least about 5.0 g, at least about 6.0 g, at least about 7.0 g, at least about 8.0 g, at least about 9.0 g, at least about 10.0 g, at least about 11 g, at least about 12 g, at least about 13 g, at least about 14 g, at least about 15 g, at least about 16 g, at least about 17 g, at least about 18 g, at least about 19 g, at least about 20 g, at least about 21 g, at least about 22 g, at least about 23 g, at least about 24 g, at least about 25 g, at least about 26 g, at least about 27 g, at least about 28 g, at least about 29 g, at least about 30 g, at least about 31 g, at least about 32 g, at least about 33 g, at least about 34 g, at least about 35 g, at least about 36 g, at least about 37 g, at least about 38 g, at least about 39 g, at least about 40 g, at least about 41 g, at least about 42 g, at least about 43 g, at least about 44 g, at least about 45 g, at least about 46 g, at least about 47 g, at least about 48 g, at least about 49 g, or at least about 50 g.

In some embodiments, the emulsion mixture comprises about 55% to about 95% (e.g., about 55% to about 90%, about 55% to about 85%, about 55% to about 80%, about 55% to about 75%, about 55% to about 70%, about 55% to about 65%, about 55% to about 60%, about 60% to about 95%, about 60% to about 90%, about 60% to about 85%, about 60% to about 80%, about 60% to about 75%, about 60% to about 70%, about 60% to about 65%, about 65% to about 95%, about 65% to about 90%, about 65% to about 85%, about 65% to about 80%, about 65% to about 75%, about 65% to about 70%, about 70% to about 95%, about 70% to about 90%, about 70% to about 85%, about 70% to about 80%, about 70% to about 75%, about 75% to about 95%, about 75% to about 90%, about 75% to about 85%, about 75% to about 80%, about 80% to about 95%, about 80% to about 90%, about 80% to about 85%, about 85% to about 95%, about 85% to about 90%, or about 90% to about 95%) by weight of the second cell mass.

In some embodiments, the emulsion mixture comprises at least 55%, at least 60%, at least 65%, at least 70%, at least 75%, at least 80%, at least 85 %, at least 90%, or at least 95% by weight of the second cell mass.

In some embodiments, hydrating the second cell mass includes adjusting the water content of the second cell mass. In some embodiments, the water content of the second cell mass is adjusted to about 75% to about 95% (e.g., about 75% to about 90%, about 75% to about 85%, about 75% to about 80%, about 80% to about 95%, about 80% to about 90%, about 80% to about 85%, about 85% to about 95%, about 85% to about 90%, or about 90% to about 95%) by weight of water.

In some embodiments, the water content of the second cell mass is at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% by weight of water. In some embodiments, the hydrated second cell mass comprises about 85% by weight of water content.

### 4.2.5. Properties of Cell Based Meat Compositions

In some embodiments, the properties of the cell based meat compositions described herein are similar or superior to other cell based meat products and/or conventional meat. In some embodiments, the one or more properties of the cell based meat compositions described herein that are similar or superior to other cell based meat products and/or conventional meat include, but are not limited to: water activity, moisture content, hardness, color, aroma, taste, chewiness, gumminess, springiness, cohesiveness, resilience, adhesiveness, protein content, lipid content, carbohydrate content, fiber content, and juiciness. The one or more properties may vary between cell based meat compositions based on the species from which the population of cells are derived, the type of food product (e.g., without limitation, patties, breasts, tenders, wings, and nuggets).

In some embodiments, the cell based meat compositions described herein include one or more of the characteristics of cell based meat compositions as described in U.S. Patent Publication No. 2021/0235733A1, which is herein incorporated by reference in its entirety.

### 4.2.5.1 Moisture Content and Water Activity

In some embodiments, a cell based meat composition comprises a moisture content about 20% to about 90% (e.g., about 20% to about 80%, about 20% to about 80%, about 20% to about 70%, about 20% to about 60%, about 20% to about 50%, about 20% to about 40%, about 20% to about 30%, about 30% to about 90%, about 30% to about 80%, about 30% to about 80%, about 30% to about 70%, about 30% to about 60%, about 30% to about 50%, about 30% to about 40%, about 40% to about 90%, about 40% to about 80%, about 40% to about 80%, about 40% to about 70%, about 40% to about 60%, about 40% to about 50%, about 50% to about 90%, about 50% to about 80%, about 50% to about 80%, about 50% to about 70%, about 50% to about 60%, about 60% to about 90%, about 60% to about 80%, about 60% to about 80%, about 60% to about 70%, about 70% to about 90%, about 70% to about 80%, or about 80% to about 90%) by weight of solution.

In some embodiments, a cell based meat composition comprises a water activity (aw) of about 0.7 a_{w} to about 0.9 a_{w} (e.g., about 0.7 a_{w} to about 0.85 a_{w}, about 0.7 a_{w} to about 0.8 a_{w}, about 0.7 a_{w} to about 0.75, about 0.75 a_{w} to about 0.9 a_{w}, about 0.75 a_{w} to about 0.85 a_{w}, about 0.75 a_{w} to about 0.8 a_{w}, about 0.8 a_{w} to about 0.9 a_{w}, about 0.8 a_{w} to about 0.8 a_{w} 5, or about 0.8 a_{w} 5 to about 0.9 a_{w}).

In some embodiments, a cell based composition comprising a water activity (a_{w}) of the cell based meat composition comprises less than or equal to 0.9 a_{w}, less than or equal to 0.8 a_{w}, less than or equal to 0.7 a_{w}, less than or equal to 0.6 a_{w}, less than or equal to 0.5 a_{w}, less than or equal to 0.4 a_{w}, less than or equal to 0.3 a_{w}, less than or equal to about 0.2 a_{w}, or less than or equal to about 0.1 a_{w}.

### 4.2.5.2 Hardness

n some embodiments, the cell based meat composition comprises a hardness of about 6000 grams (g) of force or less (e.g., about 5500 g, about 5000 g or less, about 4500 g or less, about 4000 g or less, about 3500 g or less, about 3000 g or less, about 2500 g or less, about 2000 g or less, or about 1500 g or less; between about 1500 g or less and about 6000 g or less, about 5500 g or less, about 5000 g or less, about 4500 g or less, about 4000 g or less, about 3500 g or less, about 3000 g or less, about 2500 g or less, about 2000 g or less; about 1500 g or less, about 1000 g or less, or about 500 g or less).

In some embodiments, the cell based meat composition comprises between about between about 1000 g to about 6000 g (e.g., about 1000 g to about 5500 g, about 1000 g to about 5000 g, about 1000 g to about 4500 g, about 1000 g to about 4000 g, about 1000 g to about 3500 g, about 1000 g to about 3000 g, about 1000 g to about 2500 g, about 1000 g to about 2000 g, about 1000 g to about 1500 g, about 1500 g to about 6000 g, about 1500 g to about 5500 g, about 1500 g to about 5000 g, about 1500 g to about 4500 g, about 1500 g to about 4000 g, about 1500 g to about 3500 g, about 1500 g to about 3000 g, about 1500 g to about 2500 g, about 1500 g to about 2000 g, about 2000 g to about 6000 g, about 2000 g to about 5500 g, about 2000 g to about 5000 g, about 2000 g to about 4500 g, about 2000 g to about 4000 g, about 2000 g to about 3500 g, about 2000 g to about 3000 g, about 2000 g to about 2500 g, about 2500 g to about 6000 g, about 2500 g to about 5500 g, about 2500 g to about 5000 g, about 2500 g to about 4500 g, about 2500 g to about 4000 g, about 2500 g to about 3500 g, about 2500 g to about 3000 g, about 2500 g to about 2500 g, about 3000 g to about 6000 g, about 3000 g to about 5500 g, about 3000 g to about 5000 g, about 3000 g to about 4500 g, about 3000 g to about 4000 g, about 3000 g to about 3500 g, about 3500 g to about 6000 g, about 3500 g to about 5500 g, about 3500 g to about 5000 g, about 3500 g to about 4500 g, about 3500 g to about 4000 g, about 4000 g to about 6000 g, about 4000 g to about 5500 g, about 4000 g to about 5000 g, about 4000 g to about 4500 g, about 4500 g to about 6000 g, about 4500 g to about 5500 g, about 4500 g to about 5000 g, about 5000 g to about 6000 g, about 5000 g to about 5500 g, or between about 5500 g to about 5500 g) of force.

In some embodiments,, hardness testing loads are expressed in Newton (N). In some embodiments, hardness can be expressed in kilogram-force (kgf), gram-force (gf) or (g), or pond (p). The correlation between kgf, kp, and N is: 1.0 kgf = 1,000 gf (g) = 1.0 kp = 9.81 N.

### 4.2.5.3 Resilience

In some embodiments, the cell based meat composition comprises a resilience score from a TPA analysis (see, e.g., Example 3) of about 9, about 8.5, about 8, about 7.5, about 7, about 6.5, about 6, about 5.5, about 5, about 4.5, about 4, or about 3.5, or about 3.0, or about 2.5, or about 2.0, about 1.5 or about 1.0.

In some embodiments, the cell based meat composition comprises a resilience score from a TPA analysis (see, e.g., Example 3) of between about 4.0 to about 9.0 (e.g., about 4.0 to about 8.5, about 4.0 to about 8.0, about 4.0 to about 7.5, about 4.0 to about 7.0, about 4.0 to about 6.5, about 4.0 to about 6.0, about 4.0 to about 5.5, about 4.0 to about 5.0, about 4.0 to about 4.5, about 4.5 to about 9.0, about 4.5 to about 8.5, about 4.5 to about 8.0, about 4.5 to about 7.5, about 4.5 to about 7.0, about 4.5 to about 6.5, about 4.5 to about 6.0, about 4.5 to about 5.5, about 4.5 to about 5.0, about 5.0 to about 9.0, about 5.0 to about 8.5, about 5.0 to about 8.0, about 5.0 to about 7.5, about 5.0 to about 7.0, about 5.0 to about 6.5, about 5.0 to about 6.0, about 5.5 to about 9.0, about 5.5 to about 8.5, about 5.5 to about 8.0, about 5.5 to about 7.5, about 5.5 to about 7.0, about 5.5 to about 6.5, about 5.5 to about 6.0, about 6.0 to about 9.0, about 6.0 to about 8.5, about 6.0 to about 8.0, about 6.0 to about 7.5, about 6.0 to about 7.0, about 6.0 to about 6.5, about 6.5 to about 9.0, about 6.5 to about 8.5, about 6.5 to about 8.0, about 6.5 to about 7.5, about 6.5 to about 7.0, about 7.0 to about 9.0, about 7.0 to about 8.5, about 7.0 to about 8.0, about 7.0 to about 7.5, about 7.5 to about 9.0, about 7.5 to about 8.5, about 7.5 to about 8.0, about 8.0 to about 9.0, about 8.0 to about 8.5, or about 8.5 to about 9.0).

### 4.2.5.4 Cohesiveness

In some embodiments, the cell based meat composition comprises a cohesiveness score from a TPA analysis (see, e.g., Example 3) of between about 0.1 to about 0.8 (e.g., about 0.2 to about 0.75, about 0.2 to about 0.7, about 0.2 to about 0.65, about 0.2 to about 0.6, about 0.2 to about 0.55, about 0.2 to about 0.5, about 0.2 to about 0.45, about 0.2 to about 0.4, about 0.2 to about 0.35, about 0.2 to about 0.3, about 0.2 to about 0.25, about 0.25 to about 0.8, about 0.25 to about 0.75, about 0.25 to about 0.7, about 0.25 to about 0.65, about 0.25 to about 0.6, about 0.25 to about 0.55, about 0.25 to about 0.5, about 0.25 to about 4.5, about 0.25 to about 0.4, about 0.25 to about 0.35, about 0.25 to about 0.3, about 0.3 to about 0.8, about 0.3 to about 0.75, about 0.3 to about 0.7, about 0.3 to about 0.65, about 0.3 to about 0.6, about 0.3 to about 0.55, about 0.3 to about 0.5, about 0.3 to about 0.45, about 0.3 to about 0.4, about 0.3 to about 0.35, about 0.35 to about 0.8, about 0.35 to about 0.75, about 0.35 to about 0.7, about 0.35 to about 0.65, about 0.35 to about 0.6, about 0.35 to about 0.55, about 0.35 to about 0.5, about 0.35 to about 0.45, about 0.35 to about 0.4, about 0.4 to about 0.8, about 0.4 to about 0.75, about 0.4 to about 0.7, about 0.4 to about 0.65, about 0.4 to about 0.6, about 0.4 to about 0.55, about 0.4 to about 0.5, about 0.4 to about 0.45, about 0.45 to about 0.8, about 0.45 to about 0.75, about 0.45 to about 0.7, about 0.45 to about 0.65, about 0.45 to about 0.6, about 0.45 to about 0.55, about 0.45 to about 0.5, about 0.5 to about 0.8, about 0.5 to about 0.75, about 0.5 to about 0.7, about 0.5 to about 0.65, about 0.5 to about 0.6, about 0.5 to about 0.55, about 0.55 to about 0.8, about 0.55 to about 0.75, about 0.55 to about 0.7, about 0.55 to about 0.65, about 0.55 to about 0.6, about 0.6 to about 0.8, about 0.6 to about 0.75, about 0.6 to about 0.7, about 0.6 to about 0.65, about 0.65 to about 0.8, about 0.65 to about 0.75, about 0.65 to about 0.7, about 0.7 to about 0.8, about 0.7 to about 0.75, or about 0.75 to about 0.8.

In some embodiments, the cell based meat composition comprises a cohesiveness score from a TPA analysis (see, e.g., Example 3) of about 0.80, about 0.75, about 0.7, about 0.65, about 0.6, about 0.55, about 0.5, about 0.45, about 0.4, about 0.35, about 0.3, about 0.25, about 0.2, about 0.15, about 0.1, or about 0.05.

### 4.2.5.5 Springiness

In some embodiments, the cell based meat composition comprises a springiness score from a TPA analysis (see, e.g., Example 3) of between about 20 and about 80 (e.g., about 20 to about 70, about 20 to about 60, about 20 to about 50, about 20 to about 40, about 20 to about 30, about 30 to about 80, about 30 to about 70, about 30 to about 60, about 30 to about 50, about 30 to about 40, about 40 to about 80, about 40 to about 70, about 40 to about 60, about 40 to about 50, about 50 to about 80, about 50 to about 70, about 50 to about 60, about 60 to about 80, about 60 to about 70, or about 70 to about 80.

In some embodiments, the cell based meat composition comprises a springiness score from a TPA analysis (see, e.g., Example 3) of about 80, about 75, about 70, about 65, about 60, about 55, about 50, about 45, about 40, about 35, about 30, about 25, about 20, about 15, about 10, about 5, about 1, or about 0.1.

### 4.2.5.6 Gumminess

In some embodiments, the cell based meat composition comprises a gumminess score from a TPA analysis (see, e.g., Example 3) of between about 100 to about 1000 (e.g., about 100 to about 900, about 100 to about 800, about 100 to about 700, about 100 to about 600, about 100 to about 500, about 100 to about 400, about 100 to about 300, about 100 to about 200, about 200 to about 1000, about 200 to about 900, about 200 to about 800, about 200 to about 700, about 200 to about 600, about 200 to about 500, about 200 to about 400, about 200 to about 300, about 300 to about 1000, about 300 to about 900, about 300 to about 800, about 300 to about 700, about 300 to about 600, about 300 to about 500, about 300 to about 400, about 400 to about 1000, about 400 to about 900, about 400 to about 800, about 400 to about 700, about 400 to about 600, about 400 to about 500, about 500 to about 1000, about 500 to about 900, about 500 to about 800, about 500 to about 700, about 500 to about 600, about 600 to about 1000, about 600 to about 900, about 600 to about 800, about 600 to about 700, about 700 to about 1000, about 700 to about 900, about 700 to about 800, about 800 to about 1000, about 800 to about 900, or about 900 to about 1000.

In some embodiments, the cell based meat composition comprises a gumminess score from a TPA analysis (see, e.g., Example 3) of about 5000, about 2500, about 2000, about 1500, about 1250, about 1100, about 1000, about 900, about 800, about 700, about 600, about 500, about 400, about 300, about 200, or about 100.

### 4.2.5.7 Chewiness

In some embodiments, the cell based meat composition comprises a chewiness score from a TPA analysis (see, e.g., Example 3) of between about 100 to about 500 (e.g., about 100 to about 400, about 100 to about 300, about 100 to about 200, about 200 to about 500, about 200 to about 400, about 200 to about 300, about 300 to about 500, about 300 to about 400, or about 500 to about 500).

In some embodiments, the cell based meat composition comprises a chewiness score from a TPA analysis (see, e.g., Example 3) of about 5000, about 2500, about 1500, about 1250, about 1000, about 900, about 800 about 700, about 600, about 550, about 500, about 450, about 400, about 350, about 300, about 250, about 200, about 150, about 100, about 50 or about 25.

### 4.2.5.8 Binding

In some embodiments, the cell based meat composition comprises a binding score from a TPA analysis (see Example 3 for an example binding assay) (in N^{∗}mm) at 10°C of about 500, about 400, about 300, about 200, about 190, about 180, about 170, about 160, about 150, about 140, about 130, about 120, about 110, about 100, about 90, about 80, about 70, about 60, about 50, about 40, about 30, about 20, about 10 or about 5.

In some embodiments, the cell based meat composition comprises a binding score (see Example 3 for an example binding assay) at 15°C of about 500, about 400, about 300, about 200, about 190, about 180, about 170, about 160, about 150, about 140, about 130, about 120, about 110, about 100, about 90, about 80, about 70, about 60, about 50, about 40, about 30, about 20, about 10 or about 5.

### 4.2.6. Additional Limitations

In some embodiments, the cell based meat composition does not include a cross-linking enzyme. For example, the cell based meat composition does not include one or more of glucomannan, beta-l,3-glucan, transglutaminase, calcium salts, magnesium salts, sortase, subtilisin, tyrosinase, laccase, peroxidase, and lysyl oxidase.

In some embodiments, the cell based meat composition includes one or more additional ingredients including texture modifying ingredients such as starches, modified starches, and gums, and food ingredients comprise pH regulators, anti-caking agents, colors, emulsifiers, flavors, flavor enhancers, foaming agents, anti-foaming agents, humectants, sweeteners, and other edible ingredients.

In some embodiments, the cell based meat patty comprises: about 26% by weight of water; about 7% by weight of a first cell mass; about 1.19% by weight of a salt; about 2.5% by weight of a flavoring agent; about 9.7% by weight of a first textured protein; about 11.8% by weight of a second textured protein; about 3.5% by weight of a third textured protein; about 2.5% by weight of a second flavoring agent; about 6.% by weight of an oil; about 1.8% by weight of a hydrocolloid; and about 28% by weight of a second cell mass.

### 4.3. Method of Making a Cell Based Meat Patty Comprising an Emulsion Step

Provided herein are methods for making a cell based meat composition suitable for consumption where the method includes: (a) culturing a population of cells in suspension; (b) harvesting the population of cells to produce a cell mass; (c) formulating an emulsion mixture by: (i) emulsifying an oil with a hydrocolloid to produce an oil:hydrocolloid mixture; and (ii) adding the cell mass to the oil:hydrocolloid mixture to form an emulsion mixture, and (d) forming the cell based meat composition into a patty. In some embodiments, the cell based meat patty comprises a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

In some embodiments, the step of culturing a population of cells in suspension culture comprises culturing any of the cells described herein (see Section 4.7.1) in any of the cell culture media described herein (see Section 4.7.2) in suspension culture as described herein (e.g., see Section 4.7.3).

In some embodiments, the population of cells is harvested from *in vitro* in suspension culture. In some embodiments, the population of cells is separated from the components of the suspension culture. Separation can be achieved using filtration, centrifugation, gravity, spray dry, or a combination thereof. Once separated from the components of the suspension culture (e.g., the cell culture medium), the population of cells may form a dry cell pellet or a cell powder. The cell pellet or cell powder may be hydrated with a solution to produce the hydrated first cell mass. Non-limiting examples of a solution include: water, saline solution, serum-free cell culture medium, enrichment media, and conditioned (filtered) medium from the suspension culture.

In some embodiments, forming the emulsion mixtures may require mechanical energy to form (e.g., blending, vortexing, homogenization, agitation, sonication, high pressure, or any other suitable mechanical activity). In some embodiments, when forming the emulsion mixture is aided by lower amounts of mechanical energy (e.g., agitation in a conventional mixer under moderate shear of between about 100 rpm and about 1,000 rpm ), the average droplet size of the resulting emulsion is typically larger (e.g., at least about 75% of the droplets have a diameter greater than about 25 um). In some embodiments, when the forming the emulsion mixture is aided by higher amounts of mechanical energy (e.g., homogenization in a high-pressure (e.g., about 35 bar and about 650 bar) 1-stage or 2-stage homogenizer (e.g., between about 1,000 rpm and about 10,000 rpm), or microfiuidic homogenization (between about 500 and about 2,000 bar)), the average droplet size of the resulting emulsion mixture is typically smaller (e.g., at least about 75% of the droplets have a diameter of less than about 10 um). In some embodiments, the step of forming the emulsion mixtures includes forming nanoemulsions, For example, nanoemulsions can be obtained by homogenizing in a microfluidizer. In some embodiments, where the cell based meat composition includes high lipid emulsions, the lipid is added gradually during mixing.

In some embodiments, the oil:hydrocolloid mixture is actively being mixed when the cell mass is added to the oil:hydrocolloid mixture. In some embodiments, the mixing rate of step (c)(i) at the time step (c)(ii) is performed is at least about 1 rpm (e.g., at least about 10 rpm, at least about 20 rpm, at least about 30 rpm, at least about 40 rpm, at least about 50 rpm, at least about 75 rpm, at least about 100 rpm, at least about 200 rpm, at least about 300 rpm, at least about 400 rpm, at least about 500 rpm, at least about 600 rpm, at least about 700 rpm, at least about 800 rpm, at least about 900 rpm, or at least about 1000). In some embodiments, the mixing rate of step (c)(i) is increased while the cell mass is being added to the oil:hydrocolloid mixture. In some embodiments, increasing the mixing rate of step (c)(i) includes two or more increases in the mixing rate. In some embodiments, the mixing rate of step (c)(i) at the time the cell mass is added is about at least 1 rpm (e.g., at least about 10 rpm, at least about 20 rpm, at least about 30 rpm, at least about 40 rpm, at least about 50 rpm, at least about 75 rpm, at least about 100 rpm, at least about 200 rpm, at least about 300 rpm, at least about 400 rpm, at least about 500 rpm, at least about 600 rpm, at least about 700 rpm, at least about 800 rpm, at least about 900 rpm, or at least about 1000).

In some embodiments, the oil is present in an amount of about 8% to about 24%, including values and ranges therebetween (see Section 4.2.4.1), by weight in the emulsion mixture. Non-limiting examples of oils are listed in Section 4.2.4.1. In some embodiments, the oil is soybean oil.

In some embodiments, the hydrocolloid is present in an amount of about 2.5% to about 7.5%, including values and ranges therebetween (see Section 4.2.4.2), by weight in the emulsion mixture. Non-limiting examples of hydrocolloids are listed in Section 4.2.4.2. In some embodiments, the hydrocolloid is methylcellulose.

In some embodiments, the oil:hydrocolloid mixture comprises a viscosity of about 1 cps to about 10,000 cps.

In some embodiments, the emulsion mixture comprises about 55% to about 95%, including values and ranges therebetween (see Section 4.2.4), by weight of the cell mass (e.g., the hydrated second cell mass).

In some embodiments, the emulsion mixture comprises at least 55%, at least 60%, at least 65%, at least 70%, at least 75%, at least 80%, at least 85 %, at least 90%, or at least 95% by weight of the cell mass (e.g., the hydrated second cell mass).

In some embodiments, the second cell mass comprises a population of cells harvested from cells grown in vitro in suspension culture. The population of cells is separated from the components of the suspension culture prior to hydrating the cell mass. Separation can be achieved using filtration, centrifugation, gravity, spray dry, or a combination thereof. Once separated from the components of the suspension culture (e.g., the cell culture medium), the population of cells may form a dry cell pellet or a cell powder. The cell pellet or cell powder may be hydrated with a solution to produce the hydrated first cell mass. Non-limiting examples of a solution include: water, saline solution, serum-free cell culture medium, enrichment media, and conditioned (filtered) medium from the suspension culture.

In some embodiments, hydrating the second cell mass includes adjusting the water content of the second cell mass. In some embodiments, the water content of the second cell mass is adjusted to about 75% to about 95%, including values and ranges therebetween, by weight of water. In some embodiments, the water content of the second cell mass is at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% by weight of water. In some embodiments, the hydrated second cell mass comprises about 85% by weight of water.

In some embodiments, the method includes maintaining the internal temperature of the cell mass (e.g., the second cell mass) and/or the hydrated second cell mass (e.g., the hydrated second cell mass) at a range between about 0.0°C to about 5.0°C (e.g., about 0.0°C to about 4.5°C, about 0.0°C to about 4.0°C, about 0.0°C to about 3.5°C, about 0.0°C to about 3.0°C, about 0.0°C to about 2.5°C, about 0.0°C to about 2.0°C, about 0.0°C to about 1.5°C, about 0.0°C to about 1.0°C, about 0.0°C to about 0.5°C, about 0.5°C to about 5.0°C, about 0.5°C to about 4.5°C, about 0.5°C to about 4.0°C, about 0.5°C to about 3.5°C, about 0.5°C to about 3.0°C, about 0.5°C to about 2.5°C, about 0.5°C to about 2.0°C, about 0.5°C to about 1.5°C, about 0.5°C to about 1.0°C, about 1.0°C to about 5.0°C, about 1.0°C to about 4.5°C, about 1.0°C to about 4.0°C, about 1.0°C to about 3.5°C, about 1.0°C to about 3.0°C, about 1.0°C to about 2.5°C, about 1.0°C to about 2.0°C, about 1.0°C to about 1.5°C, about 1.5°C to about 5.0°C, about 1.5°C to about 4.5°C, about 1.5°C to about 4.0°C, about 1.5°C to about 3.5°C, about 1.5°C to about 3.0°C, about 1.5°C to about 2.5°C, about 1.5°C to about 2.0°C, about 2.0°C to about 5.0°C, about 2.0°C to about 4.5°C, about 2.0°C to about 4.0°C, about 2.0°C to about 3.5°C, about 2.0°C to about 3.0°C, about 2.0°C to about 2.5°C, about 2.5°C to about 5.0°C, about 2.5°C to about 4.5°C, about 2.5°C to about 4.0°C, about 2.5°C to about 3.5°C, about 2.5°C to about 3.0°C, about 3.0°C to about 5.0°C, about 3.0°C to about 4.5°C, about 3.0°C to about 4.0°C, about 3.0°C to about 3.5°C, about 3.5°C to about 5.0°C, about 3.5°C to about 4.5°C, about 3.5°C to about 4.0°C, about 4.0°C to about 5.0°C, about 4.0°C to about 4.5°C, or about 4.5°C to about 5.0°C).

In some embodiments, the internal temperature of the cell mass (e.g., the second cell mass) and/or the hydrated cell mass (e.g., the hydrated second cell mass) remains below at least 35°C (e.g., at least below 30°C, at least below 25°C, at least below 20°C, at least below 15°C, at least below 10°C, or at least below 5°C) prior to the formulating step.

In some embodiments, the cell mass or the hydrated cell mass is not combined with a textured protein prior to the step of formulating the emulsion mixture.

In some embodiments, the emulsion mixture comprises a viscosity of a viscosity of about 1 cps to about 10,000 cps.

In some embodiments, the method also includes: forming a cell based meat patty; freezing the cell based meat patty (e.g., until the patty is solid); and coating with batter and breading.

Also provided herein is a cell based meat composition produced using the methods described in Section 4.3. In some embodiments, the cell based meat composition comprises a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

In some embodiments, the cell based meat patty comprises at least 2% by weight of oil, at least 0.5% by weight of a hydrocolloid, and at least 5% by weight of a hydrated cell mass.

### 4.4. Method of Making a Cell Based Meat Patty

Provided herein is a method of making a cell based meat composition suitable for consumption: (a) adding a hydration mixture to a textured protein to produce a hydrated textured subcomponent; wherein the hydration mixture comprises a first cell mass which comprises a population of cells harvested from suspension culture; (b) emulsifying an oil, a hydrocolloid, and a second cell mass to produce an emulsion mixture; (c) contacting the hydrated textured subcomponent with the emulsion mixture to produce a cell based meat composition; (d) forming the cell based meat composition into a patty, wherein the cell based meat patty comprises a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

Provided herein is a method of making a cell based meat composition suitable for consumption: (a) adding a hydration mixture to a textured protein to produce a hydrated textured subcomponent; wherein the hydration mixture comprises a first cell mass which comprises a population of cells harvested from suspension culture and contacting the hydrated textured subcomponent with a dry blend subcomponent; (b) emulsifying an oil, a hydrocolloid, and a second cell mass to produce an emulsion mixture; (c) contacting the hydrated textured subcomponent with the emulsion mixture to produce a cell based meat composition; (d) forming the cell based meat composition into a patty. In some embodiments, the cell based meat patty comprises a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

### 4.4.1. Adding a Hydration Mixture to a Textured Protein to Produce a Hydrated Textured Subcomponent

In some embodiments, the method of making a cell based meat composition suitable for consumption comprises a step of adding a hydration mixture to a textured protein to produce a hydrated textured subcomponent. In some embodiments, the hydration mixture comprises a first cell mass which comprises a population of cells harvested from suspension culture.

In some embodiments, the hydration mixture comprises a hydrated first cell mass which comprises a population of cells harvested from suspension culture, a water, and a flavoring agent. In some embodiments, the hydration mixture also includes a second flavoring agent.

In some embodiments, the method includes any of the hydration mixtures described herein (see Section 4.2.1). For example, in some embodiments, the hydration mixture comprises about 60% to about 80% by weight of water; about 10% to about 30% by weight of the first cell mass (e.g., any of the first cell masses described herein (see Section 4.2.1.2)); about 1.5% to about 4.5% by weight of a first flavoring agent (e.g., any of the flavoring agents described herein (see Section 4.2.1.3)); and about 3.5% to about 10.5% by weight of a second flavoring agent (e.g., any of the flavoring agents described herein (see Section 4.2.1.3)).

In some embodiments, the method includes maintaining the internal temperature of the first cell mass and/or hydrated first cell mass at a range between of about 0.0°C to about 5.0°C (e.g., about 0.0°C to about 4.5°C, about 0.0°C to about 4.0°C, about 0.0°C to about 3.5°C, about 0.0°C to about 3.0°C, about 0.0°C to about 2.5°C, about 0.0°C to about 2.0°C, about 0.0°C to about 1.5°C, about 0.0°C to about 1.0°C, about 0.0°C to about 0.5°C, about 0.5°C to about 5.0°C, about 0.5°C to about 4.5°C, about 0.5°C to about 4.0°C, about 0.5°C to about 3.5°C, about 0.5°C to about 3.0°C, about 0.5°C to about 2.5°C, about 0.5°C to about 2.0°C, about 0.5°C to about 1.5°C, about 0.5°C to about 1.0°C, about 1.0°C to about 5.0°C, about 1.0°C to about 4.5°C, about 1.0°C to about 4.0°C, about 1.0°C to about 3.5°C, about 1.0°C to about 3.0°C, about 1.0°C to about 2.5°C, about 1.0°C to about 2.0°C, about 1.0°C to about 1.5°C, about 1.5°C to about 5.0°C, about 1.5°C to about 4.5°C, about 1.5°C to about 4.0°C, about 1.5°C to about 3.5°C, about 1.5°C to about 3.0°C, about 1.5°C to about 2.5°C, about 1.5°C to about 2.0°C, about 2.0°C to about 5.0°C, about 2.0°C to about 4.5°C, about 2.0°C to about 4.0°C, about 2.0°C to about 3.5°C, about 2.0°C to about 3.0°C, about 2.0°C to about 2.5°C, about 2.5°C to about 5.0°C, about 2.5°C to about 4.5°C, about 2.5°C to about 4.0°C, about 2.5°C to about 3.5°C, about 2.5°C to about 3.0°C, about 3.0°C to about 5.0°C, about 3.0°C to about 4.5°C, about 3.0°C to about 4.0°C, about 3.0°C to about 3.5°C, about 3.5°C to about 5.0°C, about 3.5°C to about 4.5°C, about 3.5°C to about 4.0°C, about 4.0°C to about 5.0°C, about 4.0°C to about 4.5°C, or about 4.5°C to about 5.0°C).

In some embodiments, the internal temperature of the first cell mass and/or the hydrated first cell mass remains below at least 35°C (e.g., at least below 30°C, at least below 25°C, at least below 20°C, at least below 15°C, at least below 10°C, or at least below 5°C) during any of the step of the methods described herein.

In some embodiments, the method of making a cell based meat composition includes using any of the textured proteins described herein (see Section 4.2.2). For example, the method includes a textured protein which comprises a first textured protein and optionally a second textured protein. In some embodiments, the first textured protein, the second textured protein, or both are textured soy proteins (TSP).

In some embodiments, the step of adding a hydration mixture to a textured protein to produce a hydrated textured subcomponent comprises: (i) adding the hydration mixture to a first textured protein; and (ii) adding the mixture of step (i) to a second textured protein to produce the hydrated textured subcomponent. In some embodiments, the method includes maintaining the internal temperature of the mixture of step (i) at or below at least 35°C (e.g., at least below 30°C, at least below 25°C, at least below 20°C, at least below 15°C, at least below 10°C, or at least below 5°C).

In some embodiments, the water content of the hydrated textured subcomponent is provided entirely by the hydration mixture. The methods provided herein enable production of a cell based meat composition comprising the water content of the final product; no additional adjustments to water content/moisture content are needed.

In some embodiments, the first cell mass is present in the hydrated textured subcomponent at an amount of about 60% to about 95%, including values and subranges therebetween, by weight.

In some embodiments, the internal temperature of the hydrated textured subcomponent remains below at least 35°C (e.g., at least below 30°C, at least below 25°C, at least below 20°C, at least below 15°C, at least below 10°C, or at least below 5°C) during any of the methods described herein.

### 4.4.2. Adding a Dry Blend

In some embodiments, the method of making a cell based meat composition also includes contacting the hydrated textured subcomponent with a dry blend.

In some embodiments, the method includes any of the dry blends described herein (see Section 4.2.3). For example, in some embodiments, the dry blend comprises a third textured protein, a flavoring agent, or both. In some embodiments, the dry blend comprises about 58% by weight of a third textured protein and about 42% by weight of a second flavoring agent.

In some embodiments, the dry blend includes a third cell mass (see Section 4.2.3). In some embodiments, the method includes hydrating the third cell mass.

### 4.4.3. Emulsifying Step

In some embodiments, the method of making a cell based meat suitable for consumption comprises emulsifying an oil, a hydrocolloid, and a second cell mass to produce an emulsion mixture.

In some embodiments, the emulsion mixtures may require mechanical energy to form (e.g., blending, vortexing, homogenization, agitation, sonication, high pressure, or any other suitable mechanical activity). In some embodiments, when the emulsifying step is aided by lower amounts of mechanical energy (e.g., agitation in a conventional mixer under moderate shear of between about 100 rpm and about 1,000 rpm), the average droplet size of the resulting emulsion is typically larger (e.g., at least about 75% of the droplets have a diameter greater than about 25 um). In some embodiments, when the emulsifying step is aided by higher amounts of mechanical energy (e.g., homogenization in a high-pressure (e.g., between about 35 bar and about 650 bar) 1-stage or 2-stage homogenizer (e.g., between about 1,000 rpm and about 10,000 rpm], or microfiuidic homogenization (between about 500 and about 2,000 bar)), the average droplet size of the resulting emulsion mixture is typically smaller (e.g., at least about 75% of the droplets have a diameter of less than about 10 um). In some embodiments, the emulsifying step includes forming nanoemulsions, For example, nanoemulsions can be obtained by homogenizing in a microfluidizer. In some embodiments, where the cell based meat composition includes high lipid emulsions, the lipid is added gradually during mixing.

In some embodiments, the method includes any of the emulsion mixtures described herein (see Section 4.2.4). For example, in some embodiments, the emulsion mixture comprises about 8% to about 24% by weight of oil (e.g., any of the oils described herein (see Section 4.2.4.1)); about 2.5% to about 7.5% by weight of the hydrocolloid (e.g., any of the hydrocolloids described herein (see Section 4.2.4.2)); and about 55% to about 95% by weight of the second cell mass (e.g., any of the second cell masses described herein (see Section 4.2.4.3)).

In some embodiments, the emulsifying step comprises: (i) mixing an oil and a hydrocolloid; and (ii) adding a first cell mass to the mixture of step (i). In some embodiments, the oil:hydrocolloid mixture is actively being mixed when the cell mass is added to the oil:hydrocolloid mixture. In some embodiments, the mixing rate of step (i) at the time step (ii) is performed is at least about 1 rpm (e.g., at least about 10 rpm, at least about 20 rpm, at least about 30 rpm, at least about 40 rpm, at least about 50 rpm, at least about 75 rpm, at least about 100 rpm, at least about 200 rpm, at least about 300 rpm, at least about 400 rpm, at least about 500 rpm, at least about 600 rpm, at least about 700 rpm, at least about 800 rpm, at least about 900 rpm, or at least about 1000). In some embodiments, the mixing rate of step (i) is increased while the cell mass is being added to the oil:hydrocolloid mixture. In some embodiments, increasing the mixing rate of step (i) includes two or more increases in the mixing rate. In some embodiments, the mixing rate of step (i) at the time the cell mass is added is at least about 1 rpm (e.g., at least about 10 rpm, at least about 20 rpm, at least about 30 rpm, at least about 40 rpm, at least about 50 rpm, at least about 75 rpm, at least about 100 rpm, at least about 200 rpm, at least about 300 rpm, at least about 400 rpm, at least about 500 rpm, at least about 600 rpm, at least about 700 rpm, at least about 800 rpm, at least about 900 rpm, or at least about 1000).

In some embodiments, the emulsifying step is performed at temperature sufficient to form an emulsion mixture. In some embodiments, the emulsifying step is performed at temperature at or below 35°C, at or below 30°C, at or below 25°C, at or below 20°C, at or below 15°C, at or below 10°C, at or below 9°C, at or below 8°C, at or below 7 °C, at or below 6 °C, at or below 5°C, at or below 4°C, at or below 3°C, at or below 2°C, at or below 1.5°C, at or below 1°C, or at or below 0.5°C. In some embodiments, the emulsifying step is performed at our below 1.5°C.

In some embodiments, adding the cell mass (e.g., the hydrated cell mass) to the oil hydrocolloid is performed at temperature sufficient to an emulsion mixture. In some embodiments, adding the cell mass (e.g., the hydrated cell mass) to the oil hydrocolloid is performed at temperature at or below 10°C, at or below 9°C, at or below 8°C, at or below 7°C, at or below 6°C, at or below 5°C, at or below 4°C, at or below 3°C, at or below 2°C, at or below 1.5°C, at or below 1°C, or at or below 0.5°C. In some embodiments, adding the cell mass (e.g., the hydrated cell mass) to the oil hydrocolloid is performed at our below 1.5°C.

In some embodiments, the method includes maintaining the internal temperature of the second cell mass and/or hydrated second cell mass at a range between of about 0.0°C to about 5.0°C (e.g., about 0.0°C to about 4.5°C, about 0.0°C to about 4.0°C, about 0.0°C to about 3.5°C, about 0.0°C to about 3.0°C, about 0.0°C to about 2.5°C, about 0.0°C to about 2.0°C, about 0.0°C to about 1.5°C, about 0.0°C to about 1.0°C, about 0.0°C to about 0.5°C, about 0.5°C to about 5.0°C, about 0.5°C to about 4.5°C, about 0.5°C to about 4.0°C, about 0.5°C to about 3.5°C, about 0.5°C to about 3.0°C, about 0.5°C to about 2.5°C, about 0.5°C to about 2.0°C, about 0.5°C to about 1.5°C, about 0.5°C to about 1.0°C, about 1.0°C to about 5.0°C, about 1.0°C to about 4.5°C, about 1.0°C to about 4.0°C, about 1.0°C to about 3.5°C, about 1.0°C to about 3.0°C, about 1.0°C to about 2.5°C, about 1.0°C to about 2.0°C, about 1.0°C to about 1.5°C, about 1.5°C to about 5.0°C, about 1.5°C to about 4.5°C, about 1.5°C to about 4.0°C, about 1.5°C to about 3.5°C, about 1.5°C to about 3.0°C, about 1.5°C to about 2.5°C, about 1.5°C to about 2.0°C, about 2.0°C to about 5.0°C, about 2.0°C to about 4.5°C, about 2.0°C to about 4.0°C, about 2.0°C to about 3.5°C, about 2.0°C to about 3.0°C, about 2.0°C to about 2.5°C, about 2.5°C to about 5.0°C, about 2.5°C to about 4.5°C, about 2.5°C to about 4.0°C, about 2.5°C to about 3.5°C, about 2.5°C to about 3.0°C, about 3.0°C to about 5.0°C, about 3.0°C to about 4.5°C, about 3.0°C to about 4.0°C, about 3.0°C to about 3.5°C, about 3.5°C to about 5.0°C, about 3.5°C to about 4.5°C, about 3.5°C to about 4.0°C, about 4.0°C to about 5.0°C, about 4.0°C to about 4.5°C, or about 4.5°C to about 5.0°C).

In some embodiments, the internal temperature of the second cell mass and/or the hydrated second cell mass remains at least below 35°C (e.g., at least below 30°C, at least below 25°C, at least below 20°C, at least below 15°C, at least below 10°C, or at least below 5°C) during any of the methods described herein.

### 4.4.3.1 Oil

In some embodiments, the oil is present in an amount of at about 8% to about 24%, including values and ranges therebetween, by weight in the emulsion mixture. Non-limiting examples of oils are listed in Section 4.2.4.1. In some embodiments, the oil is soybean oil.

### 4.4.3.2 Hydrocolloid

In some embodiments, the hydrocolloid is present in an amount of about 2.5% to about 7.5%, including values and ranges therebetween, by weight in the emulsion mixture. Non-limiting examples of hydrocolloids are listed in Section 4.2.4.2. In some embodiments, the hydrocolloid is methylcellulose.

### 4.4.3.3 Second Cell Mass

In some embodiments, the method also includes hydrating the second cell mass. In some embodiments, the first cell mass comprises a population of cells harvested from cells grown in vitro in suspension culture. The population of cells is separated from the components of the suspension culture prior to hydrating the cell mass. Separation can be achieved using filtration, centrifugation, gravity, or a combination thereof. Once separated from the components of the suspension culture (e.g., the cell culture medium), the cells are hydrated with a solution. Non-limiting examples of a solution include: water, saline solution, serum-free cell culture medium, and conditioned (filtered) medium from the suspension culture.

In some embodiments, hydrating the first cell mass includes adjusting the water content of the second first cell mass. In some embodiments, the water content of the first cell mass is adjusted to about 75% to about 95% (e.g., about 75% to about 90%, about 75% to about 85%, about 75% to about 80%, about 80% to about 95%, about 80% to about 90%, about 80% to about 85%, about 85% to about 95%, about 85% to about 90%, or about 90% to about 95% ) by weight of water. In some embodiments, the water content of the first cell mass is at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% by weight of water. In some embodiments, the hydrated first cell mass comprises about 85% by weight of water content.

In some embodiments, the hydrated second cell mass comprises about 1.5% to about 4.5%, including values and ranges therebetween, by weight of a first flavoring agent.

In some embodiments, the hydrated second cell mass comprises about 3.5% to about 10.5%, including values and ranges therebetween, by weight of a second flavoring agent.

In some embodiments, the method includes maintaining the internal temperature of the second cell mass and/or hydrated second cell mass at a range between of about 0.0°C to about 5.0°C (e.g., about 0.0°C to about 4.5°C, about 0.0°C to about 4.0°C, about 0.0°C to about 3.5°C, about 0.0°C to about 3.0°C, about 0.0°C to about 2.5°C, about 0.0°C to about 2.0°C, about 0.0°C to about 1.5°C, about 0.0°C to about 1.0°C, about 0.0°C to about 0.5°C, about 0.5°C to about 5.0°C, about 0.5°C to about 4.5°C, about 0.5°C to about 4.0°C, about 0.5°C to about 3.5°C, about 0.5°C to about 3.0°C, about 0.5°C to about 2.5°C, about 0.5°C to about 2.0°C, about 0.5°C to about 1.5°C, about 0.5°C to about 1.0°C, about 1.0°C to about 5.0°C, about 1.0°C to about 4.5°C, about 1.0°C to about 4.0°C, about 1.0°C to about 3.5°C, about 1.0°C to about 3.0°C, about 1.0°C to about 2.5°C, about 1.0°C to about 2.0°C, about 1.0°C to about 1.5°C, about 1.5°C to about 5.0°C, about 1.5°C to about 4.5°C, about 1.5°C to about 4.0°C, about 1.5°C to about 3.5°C, about 1.5°C to about 3.0°C, about 1.5°C to about 2.5°C, about 1.5°C to about 2.0°C, about 2.0°C to about 5.0°C, about 2.0°C to about 4.5°C, about 2.0°C to about 4.0°C, about 2.0°C to about 3.5°C, about 2.0°C to about 3.0°C, about 2.0°C to about 2.5°C, about 2.5°C to about 5.0°C, about 2.5°C to about 4.5°C, about 2.5°C to about 4.0°C, about 2.5°C to about 3.5°C, about 2.5°C to about 3.0°C, about 3.0°C to about 5.0°C, about 3.0°C to about 4.5°C, about 3.0°C to about 4.0°C, about 3.0°C to about 3.5°C, about 3.5°C to about 5.0°C, about 3.5°C to about 4.5°C, about 3.5°C to about 4.0°C, about 4.0°C to about 5.0°C, about 4.0°C to about 4.5°C, or about 4.5°C to about 5.0°C).

In some embodiments, the internal temperature of the second cell mass and/or the hydrated first second mass remains below at least 35°C (e.g., at least below 30°C, at least below 25°C, at least below 20°C, at least below 15°C, at least below 10°C, or at least below 5°C) prior to the emulsifying step.

In some embodiments, the emulsion mixture comprises about 60% to about 95% by weight of the second cell mass or the hydrated second cell mass.

In some embodiments, the first cell mass or the hydrated first cell mass is not combined with a textured protein prior to the emulsifying step.

In some embodiments, the emulsion mixture comprises a viscosity of about 1 cps to about 10,000 cps.

### 4.4.4. Contacting Hydrated Subcomponent with the Emulsion Mixture

In some embodiments, the method of making a cell based meat composition suitable for consumption comprises contacting the hydrated textured subcomponent with the emulsion mixture to produce a cell based meat composition.

In some embodiments, the hydrated textured subcomponent includes any of the hydrated textured subcomponents described herein (see Section 4.4.1). For example, in some embodiments, a hydrated textured subcomponent comprises any of the hydration mixtures described herein (see Section 4.2.1) contacted with a textured protein comprising any of the textured proteins described herein (see Section 4.2.2).

In some embodiments, the emulsion mixture includes any of the emulsion mixtures described herein (see Section 4.2.4 and 4.4.3).

### 4.4.5. Forming the Cell Based Meat Composition into a Patty

In some embodiments, the method of making a cell based meat suitable for consumption comprises forming the cell based meat composition into a patty. In some embodiments, producing the cell based meat composition from the hydrated textured subcomponent and the emulsion mixture (and optionally the dry blend) may require mechanical energy (e.g., blending, vortexing, homogenization, agitation, sonication, high pressure, or any other suitable mechanical activity).

In some embodiments, the cell based meat composition produced using the methods described herein exhibit a moisture content and/or texture profile (e.g., hardness) unique from other cell based meat compositions. For example, in some embodiments, the method of making a cell based meat suitable for consumption comprises forming the cell based meat composition into a patty having a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

### 4.4.6. Temperature Controlled Environment

In some embodiments, one or more steps of the methods described herein are performed in a temperature controlled environment. In some embodiments, one or more steps of the methods described herein are performed at a temperature at or below at least 35°C (e.g., at least below 30°C, at least below 25°C, at least below 20°C, at least below 15°C, at least below 10°C, or at least below 5°C). In some embodiments, one or more steps of the methods described herein are performed at a temperature at or below at least 35°C (e.g., at least below 30°C, at least below 25°C, at least below 20°C, at least below 15°C, at least below 10°C, or at least below 5°C) in addition to the internal temperature of the first cell mass (e.g., the hydrated first cell mass), the second cell mass (e.g., the hydrated second cell mass) or both, being maintained at or below 5°C.

In some embodiments, the step of adding a hydration mixture to a textured protein to produce a hydrated textured subcomponent is performed in a temperature controlled environment where the temperature is at or below at least 35°C (e.g., at least below 30°C, at least below 25°C, at least below 20°C, at least below 15°C, at least below 10°C, or at least below 5°C).

In some embodiments, the step emulsifying step is performed in a temperature controlled environment where the temperature is at or below at least 35°C (e.g., at least below 30°C, at least below 25°C, at least below 20°C, at least below 15°C, at least below 10°C, or at least below 5°C).

In some embodiments, the step of contacting the hydrated textured subcomponent with the emulsion mixture to produce a cell based meat composition is performed in a temperature controlled environment where the temperature is at or below at least 35°C (e.g., at least below 30°C, at least below 25°C, at least below 20°C, at least below 15°C, at least below 10°C, or at least below 5°C).

### 4.4.7. Post Blending

In some embodiments, the methods of making a cell based meat composition also includes freezing the cell based meat composition.

In some embodiments, the methods of making a cell based meat composition also includes coating with batter and breading.

In some embodiments, the methods of making a cell based meat composition also includes cooking the cell based meat composition.

Provided herein is a method for making a cell based meat composition where the method includes: formulating a hydration mixture (e.g., any hydration mixtures described herein (see Section 4.2.1)), a textured protein (e.g., any textured proteins described herein (see Section 4.2.2)), a dry blend (e.g., any dry blends described herein (see Section 4.2.3)), and an emulsion mixture (e.g., any emulsion mixtures described herein (see Section 4.2.4)) into a cell based meat composition, forming the cell based meat composition into a patty, freezing the patty until solid, and coating with batter and breading.

### 4.4.8. Additional limitations

In some embodiments, the method for making a cell based meat composition does not include adding a peptide cross-linking enzyme (e.g., transglutaminase). In such embodiments, the method does not require heating the cell based meat composition, or any of its subcomponents, to a temperature at or above 40°C, which is typically required when using a cross-linking enzyme.

In some embodiments, the method for making a cell based meat composition produces a cell based meat composition comprising a water activity (aw) of about 0.7 a_{w} to about 0.9 a_{w} (see Section 4.2.5.1).

In some embodiments, the cell based meat composition produced according to the methods described herein comprises a hardness between about 1000 g and about 6000 g of force (see Section 4.2.5.2).

In some embodiments, the cell based meat composition produced according to the methods described herein comprises a resilience between about 4 and about 9 (see Section 4.2.5.3).

In some embodiments, the cell based meat composition produced according to the methods described herein comprises a cohesiveness between about 0.1 and 0.5 (see Section 4.2.5.4).

In some embodiments, the cell based meat composition produced according to the methods described herein comprises a springiness between about 20 and about 60 (see Section 4.2.5.5).

In some embodiments, the cell based meat composition produced according to the methods described herein comprises a gumminess between about 300 and 1000 (see Section 4.2.5.6).

In some embodiments, the cell based meat composition produced according to the methods described herein comprises a chewiness between about 100 and 500 (see Section 4.2.5.7).

In some embodiments, the cell based meat composition produced according to the methods described herein comprises a binding at 10°C between about 30 and about 150 (see Section 4.2.5.8) and a binding at 15°C between about 20 and about 90 (see Section 4.2.5.8).

In some embodiments, the method does not include independently modulating moisture content. In some embodiments, the moisture content is not adjusted after step (c).

### 4.5. Product by Process

Provided herein is a cell based meat composition produced by any of the methods described herein (see Section 4.3 or Section 4.4). In such embodiments, the cell based meat composition comprises a water activity (aw) of about 0.7 to about 0.9; a hardness of between about 1000 grams (g) and about 6000g; a resilience score from a TPA analysis of between about 4.0 and 9.0; a cohesiveness score from a TPA analysis of between about 0.1 to about 0.8; a springiness score from a TPA analysis of between about 20 to about 80; a gumminess score from a TPA analysis of between about 100 to about 1000; a chewiness score from a TPA analysis of between about 100 to about 500; and any of the binding scores described herein, or a combination thereof.

In some embodiments, the cell based meat composition produced by the methods described herein (see Section 4.5) comprises: an oil in an amount of at least 2% by weight, a hydrocolloid in an amount of at least 0.5% by weight; a first hydrated cell mass in an amount of at least 13% by weight; a second hydrated cell mass in an amount of least 3.5% by weight; and a textured protein present in an amount of at least 10% by weight. In some embodiments, the cell based meat composition also includes a flavoring agent in an amount of at least 2.5% by weight. In some embodiments, the cell based meat composition also includes a water in an amount of at least 13% by weight. In some embodiments, the cell based meat composition also includes batter and breading.

### 4.6. Batter and Breading

In some embodiments, the cell based meat composition includes predust, batter, breading, or a combination thereof.

In some embodiments, the batter hydration ratio (water: dry) is about 1:1, 1.1:1, 1.2:1, 1.3:1, 1.4:1, 1.5:1, 1.6:1, 1.7:1, 1.8:1, 1.9:1,2:1, 1:1.9, 1:1.8, 1:1.7, 1:1.6, 1:1.5, 1:1.4, 1:1.3, 1:1.2, or 1:1.1. In some embodiments, the batter hydration ratio (water:dry) is about 1.9:1.

In some embodiments, the methods of making a cell based meat composition also include contacting the cell based meat composition with predust, batter, breading, or a combination thereof. The contacting results in the cell based meat composition being "coated" with the predust, batter, breading, or a combination thereof. In some embodiments, contacting the cell based patty is performed under conditions sufficient to enable the cell based meat patty to be coated with at least about 10 grams (g), about 15 g, about 20 g, about 25 g, about 30 g, about 35 g, about 40 g, about 45 g, about 50 g, about 55 g, about 60 g, about 65 g, about 70 g, about 75 g, about 80 g, about 85 g, about 90 g, about 95 g, about 100 g, about 110 g, about 120 g, about 130 g, about 140 g, about 150 g, about 160 g, about 170 g, about 180 g, about 190 g, or about 200 g.

In some embodiments, predust comprises one or more ingredients selected from: a flour (e.g., fine), a protein, a textured protein (e.g., any of the textured proteins described herein (see Section 4.2.2)), and a flavoring agent (e.g., any of the flavoring agents described herein), or a combination thereof.

In some embodiments, batter comprises one or more ingredients selected from: a starch, a protein, a textured protein (e.g., any of the textured proteins described herein (see Section 4.2.2)), a flour, a flavoring agent(e.g., any of the flavoring agents described herein), and a water, or a combination thereof. In some embodiments, the batter hydration ratio (water:dry) is about 1:1, 1.1:1, 1.2:1, 1.3:1, 1.4:1, 1.5:1, 1.6:1, 1.7:1, 1.8:1, 1.9:1, 2:1, 1:1.9, 1:1.8, 1:1.7, 1:1.6, 1:1.5, 1:1.4, 1:1.3, 1:1.2, or 1:1.1.

In some embodiments, breading comprises one or more ingredients selected from: a flour, a textured protein (e.g., any of the textured proteins described herein (see Section 4.2.2)), a bread crumb, and a flavoring agent (e.g., any of the flavoring agents described herein), or a combination thereof.

### 4.7. Cell Mass/Hydrated Cell Mass

In some embodiments, the cell based meat composition comprises cells or a population of cells. In some embodiments, the cells or population of cells are harvested from *in vitro* suspension culture. In some embodiments, the cells or population of cells are harvested from adherent culture (e.g., cultivation in a cultivation infrastructure described herein).

### 4.7.1. Cells

The cell based meat compositions of the present disclosure are produced, at least in part, by the *in vitro* culturing of naturally occurring, genetically engineered, or modified animal cells in culture.

The methods provided herein are applicable to any metazoan cell in culture. Generally, the cells are from any metazoan species whose tissues are suitable for dietary consumption. In some embodiments the cells may demonstrate a capacity for differentiation into mature tissue, such as skeletal muscle tissue, other muscle tissues, or any cell, cellular bio-mass (e.g., cell mass), and/or tissue that can be consumed as meat. The cells used in the methods of the present disclosure can be primary cells, or cell lines.

In some embodiments, the cells are derived from any non-human animal species intended for human or non-human dietary consumption for example, cells of avian, ovine, caprine, porcine, bovine, piscine origin, or for example, cells of livestock, poultry, avian, game, or aquatic species).

In some embodiments, the cells are from livestock such as domestic cattle, pigs, sheep, goats, camels, water buffalo, rabbits and the like. In some embodiments, the cells are from poultry such as domestic chicken, turkeys, ducks, geese, pigeons and the like. In some embodiments, the cells are derived from chicken, duck, turkey, or a combination thereof. In some embodiments, the cells are from game species such as wild deer, gallinaceous fowl, waterfowl, hare and the like. In some embodiments, the cells are from aquatic species or semi-aquatic species harvested commercially from wild fisheries or aquaculture operations, or for sport, including certain fish, crustaceans, mollusks, cephalopods, cetaceans, crocodilians, turtles, frogs and the like.

In some embodiments, the cells are from exotic, conserved or extinct animal species. In some embodiments, the cells are from *Gallus gallus*, *Gallus domesticus*, *Bos taurus, Sous scrofa, Meleagris gallopavo, Anas platyrynchos, Salmo salar*, *Thunnus thynnus, Ovis aries, Coturnix coturnix, Capra aegagrus hircus,* or *Homarus americanus.* Accordingly, exemplary cell-based meat compositions of the present disclosure include chicken meat products, duck meat products, turkey meat products, bovine meat products, and porcine meat products.

In some embodiments, the population of cells are selected from fibroblasts, myofibroblasts, and myogenic cells, or a combination thereof.

In some embodiments, the cells are not natively myogenic (e.g., are non-myogenic cells such as fibroblasts or non-myogenic stem cells that are cultured to become myogenic cells (e.g., in suspension culture or in the cultivation infrastructure)).

In some embodiments, the cells are non-myogenic, and such non-myogenic cells can be programmed to be myogenic, for example the cells may comprise fibroblasts modified to express one or more myogenic transcription factors. In some embodiments, the myogenic transcription factors include MYOD1, MYOG, MYF5, MYF6, PAX3, PAX7, paralogs, orthologs, and genetic variants thereof. In some embodiments, the cells are modified to express one or more myogenic transcription factors as described in a PCT publication, WO/2015/066377, which is herein incorporated by reference in its entirety.

In some embodiments, the cells are genetically modified to inhibit a pathway, e.g. the HIPPO signaling pathway. Exemplary methods to inhibit the HIPPO signaling pathway as described in a PCT Application No. PCT/US2018/031276, which is herein incorporated by reference in its entirety.

In some embodiments, the cells are modified to express telomerase reverse transcriptase (TERT) and/or inhibit cyclin-dependent kinase inhibitors (CKI). In some embodiments, the cells are modified to express TERT and/or inhibit cyclin-dependent kinase inhibitors as described in a PCT publication, WO 2017/124100, which is herein incorporated by reference in its entirety.

In some embodiments, the cells are modified to express glutamine synthetase (GS), insulin-like growth factor (IGF), and/or albumin. Exemplary methods of modifying cells to express GS, IGF, and/or albumin are described in a PCT Application No. PCT/US2018/042187 which is herein incorporated by reference in its entirety.

In some embodiments, the cell-based meat product has various characteristics. Exemplary characteristics of the cell-based meat are described in U.S. Application No. 17/033,635 and PCT Application No. PCT/US2021/016681, which are herein incorporated by reference in their entireties.

In some embodiments the cells are genetically edited, modified, or adapted to grow without the need of specific ingredients including specific amino acids, carbohydrates, vitamins, inorganic salts, trace metals, TCA cycle intermediates, lipids, fatty acids, supplementary compounds, growth factors, adhesion proteins and recombinant proteins.

In some embodiments, the cells may comprise any combinations of the modifications described herein.

### 4.7.2. Cell Culture Media Formulations

In some embodiments, the cells or population of cells are grown in edible nutrient medium in adherent or suspension culture as described in U.S. Patent Publication No. 2022/0073870, which is herein incorporated by reference in its entirety.

In some embodiments, the cell culture media is itself edible, for example, it does not cause any deleterious effects if ingested. The media formulations provided herein generally comprise ingredients that are each individually or in combination edible, (e.g., safe to consume, food-grade, food-safe, food-quality, food-acceptable, food-compatible, food-category, foodstuff, for food use, eatable, comestible).

In some embodiment, an edible nutrient medium for the production of cell based meat compositions comprises a plurality of ingredients, wherein each ingredient is approved for use in food and/or is at or below its average daily intake (ADI) value. In some embodiments an intermediate version of the cell culture medium may or may not be edible. In some embodiments the cell culture medium comprises a plurality of ingredients wherein one or more ingredients is approved for use in food. In some embodiments the cell culture medium comprises a plurality of ingredients wherein each ingredient is approved for use in food. As used in the present disclosure, an ingredient "approved for use in food" refers to an ingredient that is approved for use in food by a widely accepted standard, e.g. a nationally or internationally accepted standard. These standards include, but are not limited to, standards set forth by regulatory bodies such as the Food and Drug Administration (FDA), the United States Department of Agriculture (USDA), the World Health Organization (WHO), the United Nations Food and Agriculture Organization (FAO), and the European Food Safety Authority (EFSA).

Accordingly, in some embodiments, the cell culture media of the present disclosure may comprise ingredients approved for use in food that are recognized by the FDA as Generally Recognized As Safe (GRAS). In some embodiments the cell culture media comprises ingredients that are GRAS certified ingredients, Non-GRAS certified ingredients, or mixtures thereof. In certain embodiments the cell culture media comprises only GRAS certified ingredients. As the list of GRAS substances is updated by the FDA, the GRAS certified ingredients that may be used in the formulation of the cell culture medium may be modified. In some embodiments the cell culture media comprise ingredients with a currently unknown GRAS status. It is contemplated that the media provided herein may comprise an ingredient that may currently have an unknown GRAS status or a Non-GRAS status, but in the future may be GRAS certified.

In some embodiments the cell culture media of the present disclosure may comprise ingredients approved for use in food as recognized by the FDA, the USDA, the FAO or the WHO.

In some embodiments, the cells or populations of cells are grown in media that contains no more than 10%, 9%, 8%, 7%, 6%, 5%, 4%, 3%, 2%, 1%, 0.5%, 0.1%, 0.05% or no more than 0.01%% animal-derived serum. In some embodiments, the media is substantially free of animal-derived serum or free of animal-derived serum. In some embodiments, the cell or population of cells are grown in an ACF or serum-free media. In some embodiments, the media contains no more than 10%, 9%, 8%, 7%, 6%, 5%, 4%, 3%, 2%, 1%, 0.5%, 0.1%, 0.05% or no more than 0.01%% animal-derived components. In some embodiments, the media is substantially free of animal-derived components or free of animal-derived components. In some embodiments the medium is chemically defined.

### 4.7.3. Suspension Culture

In some embodiments, the cells or populations of cells are harvested from suspension culture. In some embodiments, the population of cells are grown as batch, fed-batch, chemostat, perfusion, and/or intensified bioreactor tissue cultivator processes, or a combination thereof. In some embodiments, the suspension culture can be performed in a vessel (e.g., a fermentation tank or a bioreactor)) of a size suitable for growth of the population of cells. In some embodiments, the suspension culture system can be performed in vessel that is at least 1 liters (L), 2L, 3L, 4L, 5L, 10L, 15L, 20L, 25L, 50L, 100L, 200L, 250L, 350L, 500 liters (L), 1000L, 2,500L, 5,000L, 10,000L, 25,000L, 50,000L, 100,000L, 200,000L, 250,000L, or 500,000L.

### 4.8. Cultivation Infrastructure

In some embodiments, a cultivation infrastructure may be a tube, a cylinder, a flask, a petri-dish, a multi-well plate, a dish, a vat, a roller bottle, an incubator, a bioreactor, an industrial fermenter and the like.

Non-limiting examples of cultivation infrastructure include those described in U.S. Patent Publication Nos. 2020/0110347, 2022/0056394, and 2021/014031, which are herein incorporated by reference in their entireties.

In some embodiments, a cultivation infrastructure can be of any scale, and support any volume of cellular biomass and culturing reagents. In some embodiments, the cultivation infrastructure ranges from about 10 µL to about 100,000 L. In some embodiments, the cultivation infrastructure is about 10 µL, about 100 µL, about 1 mL, about 10 mL, about 100 mL, about 1 L, about 10 L, about 100 L, about 1000 L, about 10,000 L, or even about 100,000 L.

In some embodiments, the cultivation infrastructure comprises a substrate. In some embodiments, a cultivation infrastructure may comprise a permeable substrate (e.g. permeable to physiological solutions) or an impermeable substrate (e.g. impermeable to physiological solutions).

In some embodiments, the cultivation infrastructure comprises a primary substrate, which can be a flat, concave, or convex substrate. In some embodiments, the cultivation infrastructure further comprises a secondary substrate, either introduced, or autologous, to direct cellular growth between the substrates, e.g. to direct attachment, proliferation and hypertrophy of cells on a plane perpendicular to the primary substrate.

In some embodiments, the cultivation infrastructure comprises a hydrogel, a liquid cell culture media, or soft agar.

In some embodiments, the cultivation infrastructure does not comprise a substrate to which cells can adhere. In some embodiments, the cultivation infrastructure comprises a suspension culture, for example, supporting the growth of a self-adhering biomass, or single-cell suspension in a liquid medium.

In some embodiments, the cultivation infrastructure comprises adherent cells (i.e. those cells that adhere to a substrate). In some embodiments, the cultivation infrastructure comprises non-adherent cells (i.e. those cells that do not adhere to a substrate). In some embodiments, the cultivation infrastructure comprises both adherent and non-adherent cells.

### 5. EXAMPLES

### 5.1. Example 1: Expansion and Quality Control of Chicken Suspension Cells

### 5.1.1. Chicken Suspension Cells

Chicken fibroblast 1312 were grown in a suspension culture until the cells reached a target viable cell density (VCD), e.g. 1 million cells per milliliter. The cells were then drained from the suspension culture infrastructure and dewetted until the cells reached a target moisture content. Cells dewetted to a very low moisture content may later be rehydrated according to the methods described herein.

### 5.1.2. QC: testing for microorganisms

Safety and efficacy of the cells is checked at all stages of growth and harvesting of the cells. Cultured chicken fibroblast cells are evaluated for presence of viral, yeast, and bacterial agents. In particular, the chicken fibroblast cultures are analyzed for the presence of bacteria using protocols from the FDA's Bacteriological Analytical Manual (BAM). MycoAlertTM Mycoplasma Detection Kit is used to detect for Mycoplasms.

Total Plate Count (TPC) is synonymous with Aerobic Plate Count (APC). As indicated in Chapter 3 in the US FDA's Bacteriological Analytical Manual (BAM). Yeast and mold are analyzed according to methodology outlined in Chapter 18 of BAM Escherichia coli and coliform are analyzed according to methodology outlined in Chapter 4 of BAM. Streptococcus is analyzed using CMMEF method as described in chapter 9 of BAM. Salmonella is analyzed according to methodology outlined in Chapter 5 of BAM Viral assessment is performed by analyzing adventitious human and avian virus and bacterial agents through an Infectious Disease Polymerase Chain Reaction (PCR) performed by a third-party.

### 5.1.3. Comparison to conventional chicken

The cell based meat composition is compared to conventional chicken meat. For example, the cell based meat composition of the disclosure comprises significantly lower amounts of steroid hormones than conventional chicken mean. In another example, the cell based meat composition of the disclosure is substantially free of microbial contaminants. In another example, the cell based meat of the disclosure comprises significantly lower amounts of antibiotics as compared to conventional chicken, or is substantially free of antibiotics, or is free of antibiotics entirely. In yet another example, as compared to conventional chicken meat, the cell-based meat composition of the disclosure comprises a lower average total lipid (fat) content. In yet another example, the cell based meat composition of the disclosure comprises a similar texture profile to conventional chicken meat.

### 5.2. Example 2: Production of a chicken patty using chicken suspension cells

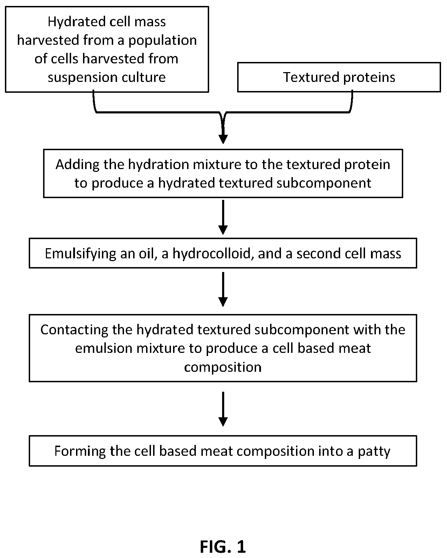

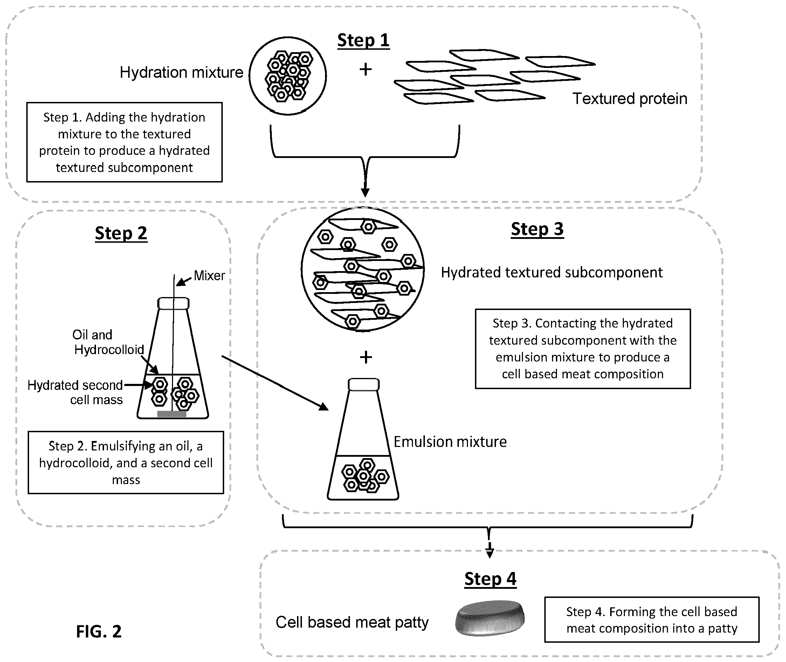

A non-limiting example of a chicken patty composition and method of making the chicken patty are provided below (see FIG. 1 and FIG. 2).

### 5.2.1. Emulsion mixture (Emulsion subcomponent)

**Table 1. Emulsion Mixture Components**

| **Emulsion Mixture** | | | |
|---|---|---|---|
| **Ingredient** | **Weight (g)** | **Percent of Emulsion Mixture** | **Percent of cell based meat patty** |
| Oil (e.g., soybean oil) | 6.0 | 16.76% | 6.0% |
| Methylcellulose (e.g., Wellence Vgegen Form 183 | 1.8 | 5.03% | 1.8% |
| Methylcellulose) | | | |
| Chicken Cell Suspension (e.g., cell mass) | 28.0 | 78.21% | 28.0% |
| Total | 35.8g | 100.00% | 35.8% |

First, oil (e.g., soybean oil) and a hydrocolloid (e.g., Methylcellulose in fine powder form) were combined and mixed (e.g., blended on level 3 for 10 seconds on a Thermomix blender^{®}) until the oil:hydrocolloid mixture formed a thin paste, reached homogeny. After reaching homogeny (e.g., mixture was the consistency of a thin paste), a hydrated first cell mass (e.g., chicken suspension cells processed according to the methods of example 1, having about 85% water by weight, and maintained at an internal temperature below about 5°C) was added to the oil:hydrocolloid mixture. In particular, while the hydrated first cell mass was being added to the oil:hydrocolloid mixture, the mixing rate was gradually increased from a low mixing speed to a high mixing speed (e.g., increased the mixing rate up to 8 on the blender by increments of 1) while material accumulating on the walls of the blender are continually scraped down to combine near the blender's blades. In total, the hydrated first cell mass was added to the oil:hydrocolloid mixture over the course of about 30 seconds.

After adding in the hydrated first cell mass to the oil:hydrocolloid mixture to create the emulsion mixture, the emulsion mixture was mixed (e.g., blended on level 8-10) for an additional 30 seconds or until the emulsion mixture color has lightened and/or the texture of the emulsion has achieved a consistency that is light, airy, fluffy, and smooth while having some bounce and jiggle. The emulsion process incorporates air into the mixture in such a way that it increased volume and lightened the color of the mixture to a light beige. The texture may be considered analogous to fire extinguisher foam or shaving cream. The textural properties of the emulsion are an important driver of binding when forming a well textured cell based meat product using suspension cells.

### 5.2.2. Hydration mixture

**Table 2. Hydration Mixture Components.**

| **Hydration Mixture** | | | |
|---|---|---|---|
| **Ingredient** | **Weight (g)** | **Percent of Hydration Mixture** | **Percent of cell based meat patty** |
| Water | 26.010 | 70.87% | 26.01% |
| Chicken Cell Suspension (e.g., cell mass) | 7.0 | 19.07% | 7.0% |
| Salt | 1.190 | 3.24% | 1.19% |
| Chicken White Meat Flavor | 2.5g | 6.81% | 2.5% |
| Total | | 100% | 36.7% |

Next, a hydration mixture was created using the ingredients listed in Table 2. The ingredients were mixed until a consistent, homogenous mixtures was obtained. Once mixed, the hydration mixture was added to the textured soy proteins (e.g., the first textured protein and the second textured protein). In particular, the first textured protein (e.g., RESPONSE^{®} 4310) was added into a vacuum sealable bag and the hydration mixture was added. After applying a vacuum but without sealing the bag, the hydration mixture and first textured protein were mixed. Next, the second textured protein (e.g., RESPONSE^{®} 4400) was added to the sealable vacuum bag containing the mixed hydration mixture and first textured protein. After applying a vacuum but without sealing the bag, the contents of the bag were mixed and let to rest at room temperature for about 15 - 20 minutes, thereby creating a hydrated textured subcomponent.

Next, the hydrated textured subcomponent was added to a mixer and mixed for 5 minutes on speed 1 with a paddle attachment.

Next, a dry blend mixture was created using the ingredients listed in Table 3.

**Table 3. Dry Blend Mixture.**

| **Dry Blend Mixture** | | | |
|---|---|---|---|
| **Ingredient** | **Weight (g)** | **Percent of Hydration Mixture** | **Percent of cell based meat patty** |
| Third Textured protein (e.g., | 3.5 | 58.33% | 3.5% |
| SuproEX 45) | | | |
| Chicken Breast Flavor | 2.5 | 41.67% | 2.5% |
| Total | | | 6.0% |

The ingredients were mixed until a consistent, homogenous mixtures was obtained. Once mixed, the dry blend mixture was added to the hydrated textured subcomponent. After adding the dry blend, the entire mixture was mixed for about 1 minute.

The emulsion mixture was then added to the hydrated textured subcomponent + dry blend mixture. The entire mixture was mixed for about 1-2 minutes to produce the cell based meat patty. The internal temperature of the cell based meat patty was then chilled to below about 1.5°C.

Next, as a non-limiting example of the type of cell based meat patties that can be made using the cell based meat patty produced in the steps described herein, 52g patties were formed and blast frozen using CO₂ (e.g., until solid).

In order to batter and/or bread the patties, the patties were allowed to temper after blast freezing. Once tempered the patties were contacted with pre-dust (e.g., a flour (e.g., fine), a protein, a textured protein (e.g., any of the textured proteins described herein (see Section 4.2.2)), and a flavoring agent (e.g., any of the flavoring agents described herein)) and agitated to remove excess pre-dust. Next, patties were contacted with batter (e.g., a starch, a protein, a textured protein (e.g., any of the textured proteins described herein (see Section 4.2.2)), a flour, a flavoring agent(e.g., any of the flavoring agents described herein), and a water, or a combination thereof a 1:1.9 dry:wet ratio) and breading (e.g., a flour, a textured protein (e.g., any of the textured proteins described herein (see Section 4.2.2)), a bread crumb, and a flavoring agent (e.g., any of the flavoring agents described herein), or a combination thereof).

The battered and breaded cell based meat patty were then processed according to the product specification. For example, for partially fried, the battered and breaded cell based meat patties were then cooked at 375°F for 35 seconds and Blast Freeze until solid.

### 5.3. Example 3: Analysis of chicken patty formulation

Chicken patties from Example 2 are analyzed according to the methods described below.

### 5.3.1. Water activity

The cell based meat patties from Example 2 are analyzed for water activity (a_{w}) using a hydgrometer (Thomas Scientific) according to the manufacturer's instructions. Hygrometers enable calculation of equilibrium relative humidity divided by 100: (a w = ERH/100) where ERH is the equilibrium relative humidity (%). Average measurements are obtained from the analysis of 3 to 5 independent samples.

Alternatively, measurement of dew points are used to determine water activity (aw). For dew point measurements, a AQUALAB 3 was using according to the manufacturer's instructions. Dew point measurements use the ratio of p (the vapor pressure of water in the sample) / Po (the saturation vapor pressure). Average measurements are obtained from the analysis of 3 to 5 independent samples.

### 5.3.2. Texture Profile Analysis (TPA)

The cell based meat patties from Example 2 are analyzed immediately after patty formation or immediately after blast freezing. When analyzing frozen patties, patties are thawed at 4°C and analyzed prior to warming to room temperature. TPA is performed using a TA.ET Express Texture Analyzer (Texture Technologies Corp.) and a polymethylmethacrylate cylinder probe of 25 mm diameter (Texture Technologies Corp., Hamilton, MA). The cylinder probe is used to compress each sample using a trigger force of 20 g to 40% compression in a 2-cycle analysis at a test speed of 0.5 mm/sec. The deformation curve of the sample is obtained, and from the deformation curve Force 1, Force2, Area FT 1:2, Time-diff 1:2, Area FT 1:3, Area FT 2:3, Area FT 4:6, and Time-diff 4:5 are derived according to the manufacturer's protocol. From this raw data, the mechanical characteristics are calculated as follows: springiness = (time-diff4:5 / time-diffl:2); cohesiveness = (area FT 4:6 / area FT 1:3); hardness = force 1; gumminess = (hardness × cohesiveness); chewiness = (springiness × gumminess); and resilience = (area FT 2:3 / area FT 1:2); as described in Food Texture and Viscosity Second Edition: Concept and Measurement, Malcolm C. Bourne, April 2002, Academic Press, New York. Average measurements are obtained from the analysis of 3 to 5 independent samples.

### 5.3.3. Binding Assay

The cell based meat patties from Example 2 are analyzed immediately after patty formation. Compression analysis is performed using a TA.XT Express Texture Analyzer (Texture Technologies Corp.) and a TA-40A polymethylmethacrylate cylinder probe of 101.6 mm diameter and 10 mm height (Texture Technologies Corp.). The test sequence features a single compression, a trigger force of 0.1 N, 60% strain, and a test speed of 0.5 mm/sec. The probe compresses the sample to 60% of its original sample height, and a deformation curve of the sample is obtained. Hardness is defined as the peak force. Binding is defined as the area under the curve between 0.2 N and the peak force. Average measurements are obtained from the analysis of 3 to 5 independent samples.

### 6. EQUIVALENTS AND INCORPORATION BY REFERENCE

All references cited herein are incorporated by reference to the same extent as if each individual publication, database entry (e.g., Genbank sequences or GeneID entries), patent application, or patent, was specifically and individually indicated incorporated by reference in its entirety, for all purposes. This statement of incorporation by reference is intended by Applicants, pursuant to 37 C.F.R. §1.57(b)(1), to relate to each and every individual publication, database entry (e.g., Genbank sequences or GeneID entries), patent application, or patent, each of which is clearly identified in compliance with 37 C.F.R. §1.57(b)(2), even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

While the invention has been particularly shown and described with reference to a preferred embodiment and various alternate embodiments, it is understood by persons skilled in the relevant art that various changes in form and details can be made therein without departing from the spirit and scope of the invention.

### 7. ADDITIONAL STATEMENTS OF INVENTION

The following numbered clauses, describing aspects of the invention, are part of the description.
1. A cell based meat composition suitable for consumption, comprising:
   (a) about 5% to about 70% by weight of a hydration mixture, wherein the hydration mixture comprises a hydrated first cell mass which comprises a population of cells harvested from suspension culture;
   (b) about 11% to about 33% by weight of a textured protein;
   (c) about 1% to about 12% by weight of a dry blend; and
   (d) about 5.0% to about 70% by weight of an emulsion mixture, wherein the emulsion mixture comprises an oil, a hydrocolloid and a hydrated second cell mass;
   wherein the cell based meat composition has a water activity (a_{w}) of about 0.7 to about 0.9 and exhibits a hardness between about 1000 g and about 6000 g.
2. The cell based meat composition of clause 1, wherein the hydrated first cell mass, the second hydrated cell mass, or both, comprises cells derived from poultry.
3. The cell based meat composition of clause 2, wherein the poultry cells are derived from chicken, duck, or turkey, or a combination thereof.
4. The cell based meat composition of any one of clauses 1-3, wherein the population of cells are selected from fibroblasts, myofibroblasts, and myogenic cells, or a combination thereof.
5. The cell based meat composition of clause 4, wherein the myogenic cells are myoblasts, myocytes, satellite cells, muscle derived stem cells, myogenic pericytes, or mesoangioblasts.
6. The cell based meat composition of any one of clauses 1-5, wherein the hydrated first cell mass, the hydrated second cell mass, or both, comprises about 1.0 ×10⁶ to about 1× 10¹¹ cells.
7. The cell based meat composition of clause 6, wherein the hydrated first cell mass, the hydrated second cell mass, or both, comprises at least about 10% packed cell volume.
8. The cell based meat composition of any one of clauses 1-7, wherein the hydrated first cell mass, comprises about 75% to about 95% by weight of water.
9. The cell based meat composition of clause 8, wherein the hydrated first cell mass comprises about 85% by weight of water content.
10. The cell based meat composition of any one of clauses 1-9, wherein the hydration mixture comprises a flavoring agent.
11. The cell based meat composition of any one of clauses 1-10, wherein the flavoring agent is selected from: chicken white meat flavor, chicken breast flavor), salt, seasoning, herbs, and flavor enhancers .
12. The cell based meat composition of any one of clauses 6-11, wherein the hydration mixture comprises:
   about 60% to about 80% by weight of water;
   about 10% to about 30% by weight of the cell mass;
   about 1.5% to about 4.5% by weight of a first flavoring agent (e.g., a salt); and
   about 3.5% to about 10.5% by weight of a second flavoring agent.
13. The cell based meat composition of any one of clauses 1-12, wherein the textured protein comprises a first textured protein and optionally a second textured protein.
14. The cell based meat composition of clause 13, wherein the first textured protein, the second textured protein, or both are textured soy proteins (TSP).
15. The cell based meat composition of clause 13 or 14, wherein the first textured protein, the second textured protein, or both are selected from a TSP having the following characteristics:
   a granule of about a 2 mm in diameter;
   a cube of about 6 mm to about 20 mm by about 6 mm to about 20 mm;
   a flake of about 2 mm in diameter by about 10 to about 70 mm in length; and
   a chunk of about 15 mm to about 25 mm in diameter by about 30 mm to about 70 mm in length,
   or a combination thereof.
16. The cell based meat composition of clause 14 or 15, wherein the first textured protein, the second textured protein, or both, comprises a water content to a textured protein ratio of about 2.60 to about 2.85.
17. The cell based meat composition of clause 14 or 15, wherein the first textured protein comprises one or more of the following characteristics: uncolored, neutral flavor, and a granule size of about 2 mm to about 6 mm.
18. The cell based meat composition of clause 14 or 15, wherein the second textured protein comprises one or more of the following characteristics: uncolored, neutral flavor, and a flake size of about 2 mm to about 6 mm.
19. The cell based meat composition of any one of clauses 1-18, wherein the oil is present in an amount of at least 8% by weight in the emulsion mixture.
20. The cell based meat composition of clause 19, wherein the oil is selected from: soybean oil, coconut oil, mango oil, sunflower oil, cottonseed oil, safflower oil, rice bran oil, cocoa butter, palm kernel oil, palm fruit oil, palm oil, rapeseed oil, canola oil, com oil, sesame oil, walnut oil, almond oil, flaxseed, jojoba oil, castor, grapeseed oil, peanut oil, olive oil, borage oil, algal oil, fungal oil, black currant oil, babassu oil, shea butter, mango butter, wheat germ oil, blackcurrant oil, sea-buckhorn oil, macadamia oil, and saw palmetto oil.
21. The cell based meat composition of clause 19, wherein the oil is soybean oil.
22. The cell based meat composition of any one of clauses 1-21, wherein the hydrocolloid is present in an amount of at least 2.5% by weight in the emulsion mixture.
23. The cell based meat composition of clause 22, wherein the hydrocolloid is selected from: methylcellulose, agar gel, gellan gum, konjac mannan, carrageenan, furcellaran, alginate, gum arabic, gum ghatti, gum tragacanth, karaya gum, guar gum, locust bean gum, tara gum, tamarind gum, inulin, arabinoxylans, b-glucans, xyloglucans, pectin, cellulose, curdlan, dextran, rhamsan gum, scleroglucan, welan gum, xanthan gum, gelatin, carboxymethylcellulose, hydroxypropyl cellulose, hydroxypropyl methyl cellulose, hydroxyethyl cellulose, propylene glycol alginate, hydroxypropyl guar, modified starches, and mixtures thereof.
24. The cell based meat composition of clause 23, wherein the hydrocolloid is methylcellulose.
25. The cell based meat composition of clause 24, wherein the hydrated second cell mass comprises a population of cells harvested from cells in suspension culture.
26. The cell based meat composition of any one of clauses 1-25, wherein the emulsion mixture comprises about 60% to about 95% by weight of the hydrated second cell mass.
27. The cell based meat composition of clause 26, wherein the hydrated second cell mass comprises about 75% to about 95% by weight of water.
28. The cell based meat composition of clause 27, wherein the hydrated second cell mass comprises about 85% by weight of water content.
29. The cell based meat composition of any one of clauses 19-28, wherein the emulsion mixture comprises:
   about 8% to about 24% by weight of oil;
   about 2.5% to about 7.5% by weight of the hydrocolloid; and
   about 55% to about 95% by weight of the second cell mass.
30. The cell based meat composition of clause 29, wherein the dry blend comprises a third textured protein, a third cell mass, a flavoring agent, or a combination thereof.
31. The cell based meat composition of clause 30, wherein the third textured protein is selected from TSP having the following characteristics:
   a granule of about 2 mm in diameter;
   a cube of about 6 mm to about 20 mm by about 6 mm to about 20 mm;
   a flake of about 2 mm in diameter by about 10 to about 70 mm in length; and
   a chunk of about 15 mm to about 25 mm in diameter by about 30 mm to about 70 mm in length),
   or a combination thereof.
32. The cell based meat composition of clause 31, wherein the third textured protein is a textured soy protein.
33. The cell based meat composition of any one of clauses 30-32, wherein the third textured is selected from TSP having the following characteristics:
   a granule of about 2 mm in diameter;
   a cube of about 6 mm to about 20 mm by about 6 mm to about 20 mm;
   a flake of about 2 mm in diameter by about 10 to about 70 mm in length; and
   a chunk of about 15 mm to about 25 mm in diameter by about 30 mm to about 70 mm in length),
   or a combination thereof.
34. The cell based meat composition of clause 30, wherein the third cell mass is hydrated.
35. The cell based meat composition of clause 30, wherein the third cell mass is not hydrated.
36. The cell based meat composition of any one of clauses 30-35, wherein the flavoring agent is selected from: chicken white meat flavor), chicken breast flavor), salt, seasoning, herbs, and flavor enhancers.
37. The cell based meat composition of any one of clauses 30-36, wherein the dry blend comprises:
   about 58% by weight of a third textured protein; and
   about 42% by weight of a second flavoring agent.
38. The cell based meat composition of any one of clauses 1-37, comprising:
   about 26% by weight of water;
   about 7% by weight of a first cell mass;
   about 1.19% by weight of a salt;
   about 2.5% by weight of a flavoring agent;
   about 9.7% by weight of a first textured protein;
   about 11.8% by weight of a second textured protein;
   about 3.5% by weight of a third textured protein;
   about 2.5% by weight of a second flavoring agent;
   about 6.% by weight of an oil;
   about 1.8% by weight of a hydrocolloid; and
   about 28% by weight of a second cell mass.
39. A method of making a cell-based meat composition suitable for consumption, comprising:
   (a) culturing a population of cells in suspension;
   (b) harvesting the population of cells to produce a cell mass;
   (c) formulating an emulsion mixture by:
      (i) emulsifying an oil with a hydrocolloid to produce an oil:hydrocolloid mixture; and
      (ii) adding the cell mass to the oil:hydrocolloid mixture to form an emulsion mixture; and
   (d) forming the cell based meat composition into a patty, wherein the patty has a water activity (aw) of about 0.7 to about 0.9 and exhibits a hardness between about 1000 g and about 6000 g.
40. The method of clause 39, wherein the population of cells are derived from poultry.
41. The method of clause 40, wherein the poultry cells are selected from chicken, duck, or turkey, or a combination thereof.
42. The method of any one of clauses 39-41, wherein the population of cells are selected from fibroblasts, myofibroblasts, and myogenic cells, or a combination thereof.
43. The method of clause 42, wherein the myogenic cells are myoblasts, myocytes, satellite cells, muscle derived stem cells, myogenic pericytes, or mesoangioblasts.
44. The method of any one of clauses 39-43, wherein harvesting comprises separating the population of cells from the components of the suspension.
45. The method of any one of clauses 39-43, wherein the cell mass comprises about 1.0 ×10⁶ to about 1× 10¹¹ cells.
46. The method of clause 45, wherein the cell mass comprises at least about 10% packed cell volume.
47. The method of any one of clauses 39-46, wherein the mixing rate of step (c)(i) at the time step (c)(ii) is performed is at least 1 rpm.
48. The method of any one of clauses 37-47, wherein the emulsifying step comprises increasing the mixing rate of step (c)(i) as the cell mass is added to the step (i) mixture.
49. The method of clause 48, wherein the increasing of the mixing rate comprises two or more increases in the mixing rate.
50. The method of any one of clauses 39-49, wherein the mixing when step (c)(ii) is performed is at 100 rpm.
51. The method of any one of clauses 39-50, wherein the oil is present in an amount of at least 8% by weight in the emulsion mixture.
52. The method of clause 51, wherein the oil is selected from: soybean oil, coconut oil, mango oil, sunflower oil, cottonseed oil, safflower oil, rice bran oil, cocoa butter, palm kernel oil, palm fruit oil, palm oil, rapeseed oil, canola oil, com oil, sesame oil, walnut oil, almond oil, flaxseed, jojoba oil, castor, grapeseed oil, peanut oil, olive oil, borage oil, algal oil, fungal oil, black currant oil, babassu oil, shea butter, mango butter, wheat germ oil, blackcurrant oil, sea-buckhorn oil, macadamia oil, and saw palmetto oil..
53. The method of clause 52, wherein the oil is soybean oil.
54. The method of any one of clauses 39-53, wherein the hydrocolloid is present in an amount of at least 2.5% by weight in the emulsion mixture.
55. The method of clause 54, wherein the hydrocolloid is selected from: methylcellulose, agar gel, gellan gum, konjac mannan, carrageenan, furcellaran, alginate, gum arabic, gum ghatti, gum tragacanth, karaya gum, guar gum, locust bean gum, tara gum, tamarind gum, inulin, arabinoxylans, b-glucans, xyloglucans, pectin, cellulose, curdlan, dextran, rhamsan gum, scleroglucan, welan gum, xanthan gum, gelatin, carboxymethylcellulose, hydroxypropyl cellulose, hydroxypropyl methyl cellulose, hydroxyethyl cellulose, propylene glycol alginate, hydroxypropyl guar, modified starches, and mixtures thereof.
56. The method of clause 55, wherein the hydrocolloid is methylcellulose.
57. The method of any one of clauses 39-56, wherein the oil:hydrocolloid mixture comprises a viscosity of about 1 cps to about 10,000 cps.
58. The method of any one of clauses 39-57, further comprising hydrating the cell mass.
59. The cell based meat composition of any one of clauses 39-58, wherein the emulsion mixture comprises about 60% to about 95% by weight of the hydrated second cell mass.
60. The method of clause 58, wherein hydrating the cell mass comprises adjusting the moisture content to a range between about 75% to about 95% by weight.
61. The method of any one of clauses 39-60, wherein the hydrated cell mass comprises a moisture content of at least 85% by weight.
62. The method of any one of clauses 39-61, further comprising maintaining the internal temperature of the cell mass and/or hydrated cell mass between about 0.0°C to about 5.0°C.
63. The method of any one of clauses 39-62, further comprising maintaining the internal temperature of the cell mass and/or hydrated cell mass between about 0.5°C to about 4.5°C.
64. The method of any one of clauses 39-62, further comprising maintaining the internal temperature of the cell mass and/or hydrated cell mass between about 1.0°C to about 4.0°C.
65. The method of any one of clauses 39-64, wherein the internal temperature of the cell mass and/or the hydrated cell mass remains below 35°C prior to the forming step.
66. The method of any one of clauses 39-65, wherein the cell mass or the hydrated cell mass is present in an amount of about 60% to about 95% by weight in the emulsion mixture.
67. The method of any one of clauses 39-66, wherein the cell mass or the hydrated cell mass is not combined with a textured protein prior to the step of formulating the emulsion mixture.
68. The method of any one of clauses 39-67, wherein the emulsion mixture comprises a viscosity of about 1 cps to about 10,000 cps.
69. The method of any one of clauses 39-68, further comprising:
   freezing the cell based meat composition until the composition is solid; and
   coating with batter and breading.
70. A cell based meat composition produced using the method of any one of clauses 39-69.
71. The cell based meat composition of clause 70, wherein the cell based meat composition has a water activity (aw) of about 0.7 to about 0.9 and/or exhibits a hardness of less than or equal to about 20 Newtons.
72. The cell based meat composition of clause 70, comprising:
   at least 2% by weight of oil,
   at least 0.5% by weight of a hydrocolloid, and
   at least 5% by weight of a hydrated cell mass.
73. A method of making a cell-based meat suitable for composition suitable for consumption, comprising:
   (a) adding a hydration mixture to a textured protein to produce a hydrated textured subcomponent; wherein the hydration mixture comprises a first cell mass which comprises a population of cells harvested from suspension culture;
   (b) emulsifying an oil, a hydrocholloid, and a second cell mass to produce an emulsion mixture;
   (c) contacting the hydrated textured subcomponent with the emulsion mixture to produce a cell based meat composition;
   (d) forming the cell based meat composition into a patty, wherein the patty has a water activity (a_{w}) of about 0.7 to about 0.9 and exhibits a hardness between about 1000 g and about 6000 g.
74. The method of clause 73, wherein the hydration mixture comprises: a water, a first cell mass, a first flavoring agent, a second flavoring agent, or a combination thereof.
75. The method of clause 74, further comprising hydrating the first cell mass.
76. The method of clause 75, wherein the hydrating the first cell mass comprises adjusting the water content of the first cell mass to a range between about 75% to about 95% by weight.
77. The method of clause 75 or 76, wherein the hydrated first cell mass comprises a water content of at least 85% by weight.
78. The method of any one of clauses 73-77, further comprising adding a first flavoring agent, a second flavoring agent, or both.
79. The method of any one of clauses 73-78, wherein the first flavoring agent, the second flavoring agent, or both, are added to the hydrated first cell mass.
80. The method of clause 79, wherein the first flavoring agent, the second flavoring agent, or both, are selected from: chicken white meat flavor), chicken breast flavor), salt, seasoning, herbs, and flavor enhancers.
81. The method of any one of clauses 80, wherein the hydration mixture comprises a flavoring agent present in an amount of at least 3.0% by weight.
82. The method of clause 80, wherein the hydration mixture comprises a salt present in an amount of at least 1.5% by weight.
83. The method of any one of clauses 73-82, further comprising maintaining the internal temperature of the first cell mass and/or the hydrated first cell mass between 0.0°C to 5°C.
84. The method of any one of clauses 73-82, further comprising maintaining the internal temperature of the first cell mass and/or the hydrated first cell mass between 0.5°C to 4.5°C.
85. The method of any one of clauses 73-82, further comprising maintaining the internal temperature of the first cell mass and/or the hydrated first cell mass between 1.0°C to 4.0°C.
86. The method of any one of clauses 73-82, wherein the internal temperature of the first cell mass and/or the hydrated first cell mass remains below 35°C during the method of any one of clauses 73-124.
87. The method of any one of clauses 73-86, wherein the hydration mixture comprises:
   about 60% to about 80% by weight of water;
   about 10% to about 30% by weight of the first cell mass;
   about 1.5% to about 4.5% by weight of a first flavoring agent (e.g., a salt); and
   about 3.5% to about 10.5% by weight of a second flavoring agent.
88. The method of any one of clauses 73-87, wherein the textured protein comprises a first textured protein and optionally a second textured protein.
89. The method of clause 88, wherein the first textured protein, the second textured protein, or both are textured soy proteins (TSP).
90. The method of clause 88 or 89, wherein the first textured protein, the second textured protein, or both are selected from TSP having the following characteristics:
   a granule of about 2 mm in diameter;
   a cube of 6 mm to about 20 mm by about 6 mm to about 20 mm;
   a flake of about 2 mm in diameter by about 10 to about 70 mm in length; and
   a chunk of about 15 mm to about 25 mm in diameter by about 30 mm to about 70 mm in length),
   or a combination thereof.
91. The method of any one of clauses 88-90, wherein the first textured protein, the second textured protein, or both, comprises a water content to a textured protein ratio of about 2.60 to about 2.85.
92. The method of any one of clauses 88-91, wherein the first textured protein comprises one or more of the following characteristics: uncolored, neutral flavor, and a granule size of about 2 mm to about 6 mm.
93. The method of any one of clauses 88-92, wherein the second textured protein comprises one or more of the following characteristics: uncolored, neutral flavor, and a flake size of about 2 mm to about 6 mm.
94. The method of any one of clauses 88-93, wherein the adding step (a) comprises:
   (i) adding the hydration mixture to a first textured protein; and
   (ii) adding the mixture of step (i) to a second textured protein to produce the hydrated textured subcomponent.
95. The method of clause 94, further comprising maintaining the internal temperature of the mixture of step (i) at or below 40°C.
96. The method of any one of clauses 73-95, wherein the moisture content of the hydrated textured subcomponent is provided entirely by the hydrated second cell mass .
97. The method of clause 96, wherein the second cell mass is present in the hydrated textured subcomponent at an amount of about 60% to about 95% by weight.
98. The method of any one of clauses 73-97, wherein the internal temperature of the hydrated textured subcomponent remains below 35°C during the method of any one of clauses 73-97.
99. The method of any one of clauses 73-98, wherein the emulsifying step comprises:
   (i) mixing an oil and a hydrocholloid; and
   (ii) adding a second cell mass to the mixture of step (i).
100. The method of clause 99, wherein the mixing rate of step (i) at the time step (ii) is performed is at least 1 rpm.
101. The method of clause 99, wherein the emulsifying step comprises increasing the mixing rate of step (i) as the second cell mass is added to the step (i) mixture.
102. The method of clause 101, wherein the increasing of the mixing rate of step (i) comprises two or more increases in the mixing rate.
103. The method of any one of clauses 73-102, wherein the oil is present in an amount of at least 8% by weight in the emulsion mixture.
104. The method of clause 103, wherein the oil is selected from: soybean oil, coconut oil, mango oil, sunflower oil, cottonseed oil, safflower oil, rice bran oil, cocoa butter, palm kernel oil, palm fruit oil, palm oil, rapeseed oil, canola oil, com oil, sesame oil, walnut oil, almond oil, flaxseed, jojoba oil, castor, grapeseed oil, peanut oil, olive oil, borage oil, algal oil, fungal oil, black currant oil, babassu oil, shea butter, mango butter, wheat germ oil, blackcurrant oil, sea-buckhorn oil, macadamia oil, and saw palmetto oil.
105. The method of clause 104, wherein the oil is soybean oil.
106. The method of any one of clauses 73-105, wherein the hydrocolloid is present in an amount of at least 2.5% by weight in the emulsion mixture.
107. The method of clause 106, wherein the hydrocolloid is selected from: methylcellulose, agar gel, gellan gum, konjac mannan, carrageenan, furcellaran, alginate, gum arabic, gum ghatti, gum tragacanth, karaya gum, guar gum, locust bean gum, tara gum, tamarind gum, inulin, arabinoxylans, b-glucans, xyloglucans, pectin, cellulose, curdlan, dextran, rhamsan gum, scleroglucan, welan gum, xanthan gum, gelatin, carboxymethylcellulose, hydroxypropyl cellulose, hydroxypropyl methyl cellulose, hydroxyethyl cellulose, propylene glycol alginate, hydroxypropyl guar, modified starches, and mixtures thereof.
108. The method of clause 107, wherein the hydrocolloid is methylcellulose.
109. The method of any one of clauses 73-108, further comprising hydrating the second cell mass.
110. The cell based meat composition of any one of clauses 73-109, wherein the emulsion mixture comprises about 60% to about 95% by weight of the hydrated second cell mass.
111. The method of clause 109, wherein hydrating the second cell mass comprises adjusting the water content of the first cell mass to a range between about 75% to about 95% by weight of water.
112. The method of clause 111, wherein the hydrated second cell mass comprises a water content of at least 85% by weight of water.
113. The method of any one of clauses 73-112, further comprising maintaining the internal temperature of the second cell mass and/or the hydrated second cell mass between about 0.0°C to about 5.0°C.
114. The method of any one of clauses 73-112, further comprising maintaining the internal temperature of the second cell mass and/or the hydrated second cell mass between about 0.5°C to about 4.5°C.
115. The method of any one of clauses 73-112, further comprising maintaining the internal temperature of the second cell mass and/or the hydrated second cell mass between about 1.0°C to about 4.0°C.
116. The method of any one of clauses 73-115, wherein the internal temperature of the second cell mass and/or the hydrated second cell mass remains below 35°C prior to the emulsifying step.
117. The method of any one of clauses 73-116, wherein the emulsion mixture comprises about 55% to about 95% by weight of the second cell mass or the hydrated second cell mass.
118. The method of any one of clauses 73-117, wherein the second cell mass or the hydrated second cell mass is not combined with a textured protein prior to the emulsifying step.
119. The method of any one of clauses 73-118, wherein the emulsion mixture comprises a viscosity of about 1 cps to about 10,000 cps.
120. The method of any one of clauses 73-119, wherein the emulsion mixture comprises:
   about 8% to about 24% by weight of oil;
   about 2.5% to about 7.5% by weight of the hydrocolloid; and
   about 55% to about 95% by weight of the second cell mass.
121. The method of any one of clauses 73-120, further comprising contacting the hydrated textured subcomponent with a dry blend mixture.
122. The method of clause 121, wherein the dry blend comprises a third textured protein, a flavoring agent, or both.
123. The method of clause 122, wherein the third textured protein is selected from:
   a granule of about 2 mm in diameter;
   a cube of about 6 mm to about 20 mm by about 6 mm to about 20 mm;
   a flake of about 2 mm in diameter by about 10 to about 70 mm in length; and
   a chunk of about 15 mm to about 25 mm in diameter by about 30 mm to about 70 mm in length),
   or a combination thereof.
124. The method of clause 122 or 123, wherein the third textured protein comprises a water content to a textured protein ratio of about 2.60 to about 2.85.
125. The method of any one of clauses 122-124, wherein the textured protein comprises a textured soy protein (TSP).
126. The method of clause 125, wherein the third textured is selected from TSP having the following characteristics:
   a granule of about 2 mm in diameter;
   a cube of about 6 mm to about 20 mm by about 6 mm to about 20 mm;
   a flake of about 2 mm in diameter by about 10 to about 70 mm in length; and
   a chunk of about 15 mm to about 25 mm in diameter by about 30 mm to about 70 mm in length),
   or a combination thereof.
127. The method of clause 121, wherein the dry blend comprises a third cell mass, a flavoring agent, or both.
128. The method of clause 127, wherein the third cell mass is hydrated.
129. The method of clause 127, wherein the third cell mass is not hydrated.
130. The method of any one of clauses 122-129, wherein the dry blend comprises two or more additional flavoring agents.
131. The method of any one of clauses 121-130, wherein the flavoring agent or additional flavoring agents are selected from: chicken white meat flavor, chicken breast flavor), salt, seasoning, herbs, and flavor enhancers.
132. The method of any one of clauses 73 -131, wherein the cell mass, the hydrated first cell mass, the second cell mass, the hydrated second cell mass, or a combination thereof, comprises cells derived from poultry.
133. The method of clause 132, wherein the poultry cells are derived from: chicken, duck, or turkey, or a combination thereof.
134. The method of clause 133, wherein the cells are selected from fibroblasts, myofibroblasts, and myogenic cells, or a combination thereof.
135. The method of clause 134, wherein the myogenic cells are myoblasts, myocytes, satellite cells, muscle derived stem cells, mesenchymal stem cells, myogenic pericytes, or mesoangioblasts.
136. The method of any one of clauses 132-135, wherein the cells are grown *in vitro* in suspension culture.
137. The method of clause 136, further comprising:
   harvesting the cells from suspension culture; and
   separating the population of cells from the components of the suspension culture prior to hydrating the cell mass.
138. The method of any one of clauses 73-137, wherein the first cell mass, the hydrated first cell mass, the second cell mass, the hydrated second cell mass, or a combination thereof, comprises about 1.0 ×10⁶ to about 1× 10¹¹ cells.
139. The method of clause 138, wherein the first cell mass, the hydrated first cell mass, the second cell mass, the hydrated second cell mass, or a combination thereof, comprises at least about 10% percent viable cells.
140. The method of any one of clauses 73-139, wherein the method does not include adding a peptide cross-linking enzyme (e.g., transglutaminase).
141. The method of any one of clauses 73-140, wherein the cell based meat composition has a water activity (a_{w}) of about 0.7 to about 0.9 .
142. The method of any one of clauses 73-140, wherein the moisture content of the cell based meat composition comprises less than or equal to 0.8 a_{w}.
143. The method of any one of clauses 73-141, wherein the method does not include independently modulating moisture content.
144. The method of clause 143, wherein the moisture content is not adjusted after step (c).
145. The method of any one of clauses 73-144, further comprising freezing the cell based meat composition.
146. The method of clause 145, further comprising coating with batter and breading.
147. The method of any one of clauses 73-146, further comprising cooking the cell based meat composition.
148. A cell based meat composition produced by any of the method of any one of clauses 73-147.
149. The cell based meat composition of clause 148, comprising:
   an oil in an amount of at least 2% by weight;
   a hydrocolloid in an amount of at least 0.5% by weight;
   a first hydrated cell mass in an amount of at least 13% by weight;
   a second hydrated cell mass in an amount of least 3.5% by weight; and
   a textured protein present in an amount of at least 10% by weight.
150. The cell based meat composition of clause 149, comprising:
   a flavoring agent in an amount of at least 2.5% by weight.
151. The cell based meat composition of clause 150, comprising:
   a water in an amount of at least 13% by weight.

The cell based meat composition of clause 149, further comprising batter and breading.

## Claims

1. A method of making a cell-based meat composition suitable for consumption, comprising:
(a) adding a hydration mixture to a textured protein to produce a hydrated textured subcomponent; wherein the hydration mixture comprises a hydrated first cell mass which comprises a population of cells harvested from suspension culture;
(b) emulsifying an oil, a hydrocholloid, and a hydrated second cell mass to produce an emulsion mixture, wherein the hydrated second cell mass comprises a second population of cells harvested from suspension culture;
(c) contacting the hydrated textured subcomponent with the emulsion mixture to produce a cell based meat composition suitable for consumption; and
(d) forming the cell based meat composition suitable for consumption into a patty.

2. A method of making a cell-based meat composition suitable for consumption, comprising:
(a) culturing a population of cells in suspension;
(b) harvesting the population of cells to produce a cell mass;
(c) formulating an emulsion mixture by:
(i) emulsifying an oil with a hydrocolloid to produce an oil:hydrocolloid mixture; and
(ii) adding the cell mass to the oil:hydrocolloid mixture to form an emulsion mixture; and
(d) forming the emulsion mixture into a cell-based meat product.

3. The method of claim 1 or 2, wherein the population of cells, the second population of cells, or both, are derived from poultry cells having phenotypes of fibroblasts, myofibroblasts, and myogenic cells, or a combination thereof.

4. The method of any one of claims 1-3, wherein one or more of the hydrated first cell mass, the hydrated second cell mass, or the cell mass comprises about 1.0 ×10⁶ to about 1× 10¹¹ cells.

5. The method of any one of claims 1-4, wherein one or more of the hydrated first cell mass, the hydrated second cell mass, or the cell mass, comprises at least about 10% packed cell volume.

6. The method of any one of claims 1-5, further comprising maintaining the internal temperature of one or more of the hydrated first cell mass, the hydrated second cell mass, or the cell mass between about 0.0°C to about 5.0°C.

7. The method of any one of claims 1-5, wherein the internal temperature of one or more of the hydrated first cell mass, the hydrated second cell mass, or the cell mass remain below 35°C prior to the forming step.

8. The method of any one of claims 1-7, wherein the oil is present in an amount of at least 8% by weight in the emulsion mixture.

9. The method of claim 8, wherein the oil is selected from: soybean oil, coconut oil, mango oil, sunflower oil, cottonseed oil, safflower oil, rice bran oil, cocoa butter, palm kernel oil, palm fruit oil, palm oil, rapeseed oil, canola oil, com oil, sesame oil, walnut oil, almond oil, flaxseed, jojoba oil, castor, grapeseed oil, peanut oil, olive oil, borage oil, algal oil, fungal oil, black currant oil, babassu oil, shea butter, mango butter, wheat germ oil, blackcurrant oil, sea-buckhorn oil, macadamia oil, and saw palmetto oil.

10. The method of any one of claims 1-9, wherein the hydrocolloid is present in an amount of at least 2.5% by weight in the emulsion mixture.

11. The method of claim 10, wherein the hydrocolloid is selected from: methylcellulose, agar gel, gellan gum, konjac mannan, carrageenan, furcellaran, alginate, gum arabic, gum ghatti, gum tragacanth, karaya gum, guar gum, locust bean gum, tara gum, tamarind gum, inulin, arabinoxylans, b-glucans, xyloglucans, pectin, cellulose, curdlan, dextran, rhamsan gum, scleroglucan, welan gum, xanthan gum, gelatin, carboxymethylcellulose, hydroxypropyl cellulose, hydroxypropyl methyl cellulose, hydroxyethyl cellulose, propylene glycol alginate, hydroxypropyl guar, modified starches, and mixtures thereof.

12. The method of any one of claims 1-11, further comprising:
freezing the cell based meat composition until the composition is solid; and
coating with batter and breading.

13. A cell based meat composition produced using the method of any one of claims 1-12, comprising:
at least 2% by weight of oil,
at least 0.5% by weight of a hydrocolloid, and
at least 5% by weight of a hydrated second cell mass or cell mass.

14. A cell based meat composition suitable for consumption, comprising:
(a) about 5% to about 70% by weight of a hydration mixture, wherein the hydration mixture comprises a hydrated first cell mass which comprises a population of cells harvested from suspension culture;
(b) about 11% to about 33% by weight of a textured protein;
(c) about 1% to about 12% by weight of a dry blend; and
(d) about 5.0% to about 70% by weight of an emulsion mixture, wherein the emulsion mixture comprises an oil, a hydrocolloid and a hydrated second cell mass, wherein the hydrated second cell mass comprises a second population of cells harvested from suspension culture;
wherein the cell based meat composition has a water activity (aw) of about 0.7 to about 0.9 and exhibits a hardness between about 1000 g and about 6000 g.

15. The cell based meat composition claim 14, wherein the hydrated first cell mass, the hydrated second cell mass, or both, comprise at least about 10% packed cell volume.

16. The cell based meat composition of claim 14 or 15, wherein the population of cells, the second population of cells, or both are derived from poultry cells having phenotypes of fibroblasts, myofibroblasts, and myogenic cells, or a combination thereof.
